(19) Europäisches Patentamt
European Patent Office
Office européen des brevets

(11) Veröffentlichungsnummer: **0 029 003**
**B1**

(12) # EUROPÄISCHE PATENTSCHRIFT

(45) Veröffentlichungstag der Patentschrift: **05.12.84**

(51) Int. Cl.³: **D 06 L 3/12**, C 07 F 9/09

(21) Anmeldenummer: **80810324.6**

(22) Anmeldetag: **27.10.80**

(54) **Salze kationischer Aufheller, deren Herstellung und deren Verwendung auf organischen Materialien sowie deren konzentrierte wässrige Lösungen.**

(30) Priorität: **01.11.79 CH 9808/79**

(43) Veröffentlichungstag der Anmeldung: **20.05.81 Patentblatt 81/20**

(45) Bekanntmachung des Hinweises auf die Patenterteilung: **05.12.84 Patentblatt 84/49**

(84) Benannte Vertragsstaaten: **CH DE FR GB IT LI**

(56) Entgegenhaltungen:
**EP-A-0 001 620**
**AU-D-2 294 367**
**FR-A-2 012 166**
**FR-A-2 111 451**
**FR-A-2 318 910**
**GB-A-1 351 489**

(73) Patentinhaber: **CIBA-GEIGY AG, Postfach, CH-4002 Basel (CH)**

(72) Erfinder: **Meyer, Hans Rudolf, Dr., Bollwerkstrasse 102, CH-4102 Binningen (CH)**

**EP 0 029 003 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue kationische optische Aufheller, ein Verfahren zu deren Herstellung sowie deren Verwendung zum optischen Aufhellen von organischen Materialien sowie konzentrierte wässrige Lösungen der optischen Aufheller.

Es ist in der Literatur eine grössere Zahl kationischer Aufheller bekannt, die bei Raumtemperatur in Wasser nur beschränkt löslich sind. Solche Aufheller sind quaternierte Ammoniumsalze basischer Aufheller, die z.B. in den Patentschriften beschrieben sind, welche meist auch die basischen Aufheller selbst umfassen. Solche Verbindungen entsprechen dem weiter unten angegebenen Aufhellerteil $B_1$.

Aus der FR-A-2 111 451 sind auch 1,3-Diphenylpyrazoline mit einer quaternären Ammoniumgruppe bekannt, die, falls die Ammoniumgruppe eine unsubstituierte oder substituierte Hydroxylalkylgruppe aufweist, in Wasser und in organischen polaren Lösungsmitteln eine erhöhte Löslichkeit zeigen.

Die Erfindung betrifft nun neue kationische Aufheller der Formel

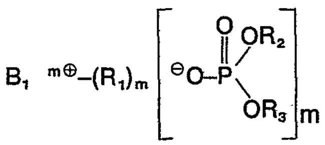

worin $B_1$ einen Aufheller aus der Gruppe der 2-Furanyl-benzimidazole, 2-Azolyl-benzimidazole, 2-Stilbenyl-benzimidazole, basischen 1,2-Bis-(azolyl)-äthylene, 2,5-Bis-(benzimidazolyl)-furane, basischen 4,4'-Bis-(azolyl)-stilbene, basischen 2-Phenyl-5-azolyl-thiophene, basischen 4,4'-Distyryl-biphenyle, basischen 1,4-Distryryl-benzole, basischen 3,7-disubstituierten Cumarine, basischen Naphthalimide, basischen 4,4'-Triazinylamino-stilbene, basischen 2-Stilben-4-yl-naphthotriazole und basischen Triazolyl- oder Pyrazolylstilbene, m die Anzahl basischer Aminogruppen und $R_1$, $R_2$ und $R_3$ unsubstituiertes oder nicht-chromophor substituiertes Alkyl mit 1 bis 4 C-Atomen oder unsubstituiertes oder nicht-chromophor substituiertes Alkenyl mit 2 bis 4 C-Atomen bedeuten.

Die nicht quaternierten Vertreter von $B_1$ sind wie folgt z.B. bekannt: die 2-Furanyl-benzimidazole aus US-A-3 497 525; 3 631 168; 3 637 734; 3 900 419; 4 009 994; 4 018 789 und 4 146 725; DE-A-2 346 316; 2 807 008; 2 852 531 und 2 821 116, die 2-Azolylbenzimidazole aus US-A-4 083 958, die 2-Stilbenyl-benzimidazole aus US-A-3 133 916, basische 1,2-Bis-(azolyl)-äthylene aus US-A-2 808 407; 3 259 619; 3 169 960, 2,5-Bis-(benzimidazolyl)-furane aus US-A-3 005 779, basische 4,4'-Bis-(azolyl)-stilbene aus US-A-3 583 984, basische 2-Phenyl-5-azolyl-thiophene aus US-A-3 264 315, basische 4,4'-Distyryl-biphenyle aus US-A-3 984 399, basische 1,4-Distyryl-benzole aus US-A-3 755 446, basische 3,7-disubstituierte Cumarine aus US-A-3 251 851; 3 271 412; 3 625 952; 3 663 560; 4 005 098; DE-A-1 919 181, basische Naphthalimide aus GB-A-962 019; 1 227 239; US-A-3 697 525; 3 625 947; 3 880 859; 3 941 791; 3 776 932; 4 075 221; DE-A-2 064 159; 2 507 459; 2 641 001, basische 4,4'-Bis-triazinylamino-stilbene aus DE-A-1 930 309; 2 060 085, basische 2-Stilben-4-yl-naphtho-triazole aus DE-B-1 090 169 und basische 4,4'-Bis-(triazolyl)- und -(pyrazolyl)-stilbene aus US-A-3 824 236; 3 796 706.

Die neuen kationischen Aufheller zeichnen sich gegenüber jenen der genannten Veröffentlichungen durch eine bei Raum- und höherer Temperatur stark verbesserte Löslichkeit in Wasser und in polaren organischen Lösungsmitteln aus. Diese verbesserte Löslichkeit ermöglicht die Herstellung von Aufhellerlösungen mit Konzentrationen bis zu 60 Gewichtsprozent, vorzugsweise von 10 bis 50 Gewichtsprozent Aufhellersubstanz.

Die Anzahl basischer Aminogruppen beträgt vorzugsweise 1 oder 2. Es können aber auch noch weitere vorhanden sein.

Von Bedeutung sind kationische Aufheller der Formel

(2) 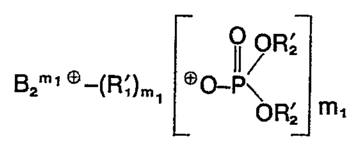

worin $m_1$ 1 oder 2 bedeutet und $R'_1$ und $R'_2$ Alkyl mit 1 bis 4 C-Atomen oder Benzyl und $B_2$ einen Aufheller der Formel

(3) 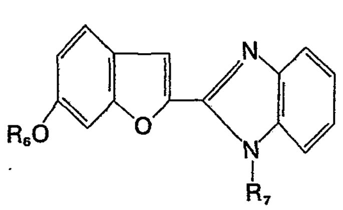 ,

(4) 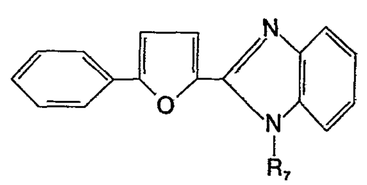 ,

(5) 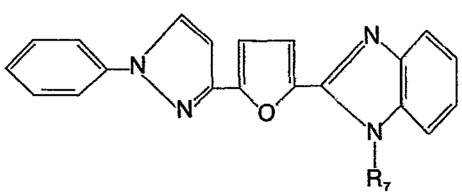 ,

(6) 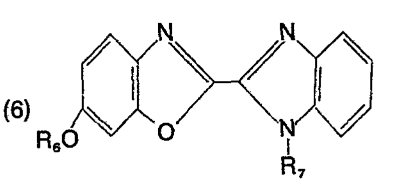 ,

(7) 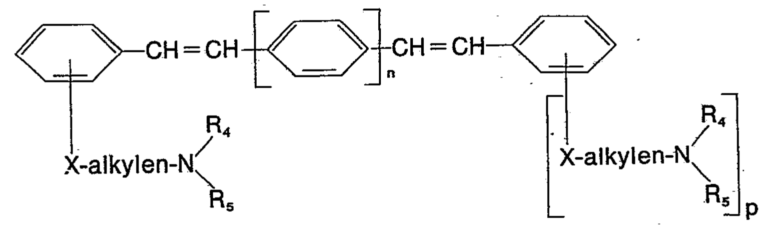

(8) 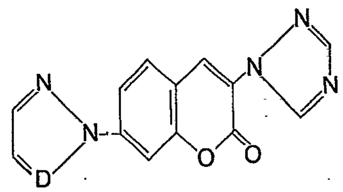

oder

(9) 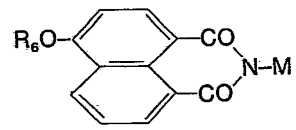

worin M Alkylen-N($R_4$)($R_5$), Pyrazolyl oder Pyridinyl, $R_4$ und $R_5$ unabhängig voneinander unsubstituiertes oder nicht-chromophor substituiertes Alkyl mit 1 bis 6 C-Atomen, unsubstituiertes oder nicht-chromophor substituiertes Alkenyl mit 2 bis 6 C-Atomen oder $R_4$ und $R_5$ zusammen mit dem N-Atom auch einen 5- bis 7-gliedrigen heterocyclischen Ring, $R_6$ und $R_7$ unabhängig voneinander unsubstituiertes oder nicht-chromophor substituiertes Alkyl mit 1 bis 6 C-Atomen, unsubstituiertes oder nicht-chromophor substituiertes Alkenyl mit 2 bis 6 C-Atomen oder Phenyl, D Stickstoff oder die CH-Gruppe, X ein nicht-chromophores Brückenglied oder die direkte Bindung, n die Zahl 1 oder 2 und p die Zahl 0 oder 1 bedeuten, wobei die Benzol- und die heterocyclischen Ringe auch nicht-chromophor substituiert sein können.

Als nicht-chromophore Brückenglieder X kommen Sauerstoff, Schwefel, $-SO_2N(R)-$, $-CON(R)-$, $-OCO-$, $-COO-$, die direkte Bindung, $-O-C_{1-3}$-Alkylen$-CON(R)-$ oder $-O-C_{1-3}$-Alkylen$-COO-$ in Betracht, worin R für Wasserstoff oder Alkyl mit 1 bis 6 C-Atomen steht, das substituiert sein kann oder zusammen mit $R_4$ einen Piperazinring bildet.

Die «Alkylen»-Reste können verschiedenartig geradkettig oder verzweigt sein und 1 bis 12 und besonders 1 bis 6 C-Atome aufweisen. Sie können auch eine Hydroxygruppe tragen.

Geeignete Alkylreste $R_4$ und $R_5$ sind solche mit 1 bis 6, vorzugsweise 1 bis 4 C-Atomen. Sie können beispielsweise mit Halogen, Cyano, Hydroxy, Alkoxy, Phenyl oder Alkoxycarbonyl mit 1 bis 4 C-Atomen substituiert sein. Die Reste $R_4$ und $R_5$ können zusammen einen 5- bis 7-gliedrigen heterocyclischen Ring bilden, z.B. einen Piperidin-, Pyrrolidin-, Hexamethylenimin- oder Morpholinring, der durch Alkylgruppen mit 1 bis 4 C-Atomen substituiert sein kann. $R_4$ kann ferner mit R zusammen einen Piperazinring bilden.

Unter nicht-chromophor substituierten Alkylgruppen sind für $R_6$ besonders die Alkoxyalkylgruppe mit insgesamt 3 bis 6 Kohlenstoffatomen und die Benzylgruppe zu verstehen und für $R_7$ mit Hydroxy, Alkoxy mit 1 bis 4 C-Atomen, Carbalkoxy mit 2 bis 5 C-Atomen, Cyano, Phenyl oder Carbamoyl substituiertes Alkyl zu verstehen.

Die für $R_6$ und $R_7$ stehenden Alkenylgruppen sind vorzugsweise unsubstituiert und enthalten vorzugsweise 3 bis 4 Kohlenstoffatome und die Phenylreste können z.B. noch mit 1 bis 2 Alkylgruppen mit je 1 bis 4 C-Atomen, Chlor oder Alkoxy mit 1 bis 4 Kohlenstoffatomen substituiert sein.

Als nicht-chromophore Substituenten der Benzol- oder heterocyclischen Kerne seien beispielsweise erwähnt: Halogenatome, Alkylgruppen mit 1 bis 6 C-Atomen, Cyclohexylgruppen, Alkenylgruppen mit 3 bis 6 C-Atomen, Alkoxygruppen mit 1 bis 6 C-Atomen, Alkenyloxygruppen mit 3 bis 6 C-Atomen, Phenoxy, das noch mit Methyl, Chlor oder Methoxy substituiert sein kann, Alkylsulfonylgruppen mit 1 bis 6 C-Atomen, Phenylsulfonyl- oder Phenoxy-sulfonylgruppen, die noch mit Methyl, Chlor oder Methoxy substituiert sein können, Benzylsulfonyl, Cyano, Trifluormethyl, Alkoxycarbonyl mit 2 bis 7 C-Atomen, Carboxy, Phenyl, Benzyl, $-CONZ_1Z_2$ oder $-SO_2NZ_1Z_2$, worin $Z_1$ für Wasserstoff, Alkyl mit 1 bis 4 Kohlenstoffatomen, Alkenyl mit 3 oder 4 Kohlenstoffatomen, Hydroxyalkyl mit 2 bis 4 Kohlenstoffatomen, Cyclohexyl, Alkoxyalkyl mit insgesamt 3 bis 6 Kohlenstoffatomen, Benzyl oder Dialkylaminoalkyl mit insgesamt 3 bis 7 Kohlenstoffatomen, welches durch $R'_1$ quaterniert sein kann, $Z_2$ für Wasserstoff, Alkyl mit 1 bis 4 Kohlenstoffatomen oder Alkenyl mit 3 oder 4 Kohlenstoffatomen und $Z_1$ und $Z_2$ zusammen mit dem Stickstoffatom an das sie gebunden sind für einen Pyrrolidin-, Piperidin-, N-Alkylpiperazin- mit 1 bis 3 Kohlenstoffatomen im Alkylteil, Hexamethylenimin oder Morpholinring stehen.

In Formel (2) bedeuten $R'_1$ und $R'_2$ vorzugsweise Alkyl mit 1 bis 3 Kohlenstoffatomen, $R_4$, $R_5$ und $R_6$ sind vorzugsweise unsubstituierte Alkylreste mit 1 bis 4 Kohlenstoffatomen, wobei $R_4$ und $R_5$ gegebenenfalls zusammen mit dem Stickstoffatom, an das sie gebunden sind, auch einen Pyrrolidin-, Piperidin-, Hexamethylenimin- oder Morpholinrest bilden können. $R_4$ kann auch zusammen mit R einen Piperazinring bilden. $R_7$ ist vorzugsweise Alkyl mit 1 bis 4 Kohlenstoffatomen, Alkenyl mit 3 oder 4 Kohlenstoffatomen, Cyanoalkyl mit 2 oder 3 Kohlenstoffatomen, Carbalkoxyalkyl mit 1 bis 3 Kohlenstoffatomen im Alkoxyteil, Benzyl oder Phenyl. D steht vorzugsweise für $=N-$, X für

Sauerstoff, die direkte Bindung, $-SO_2N(R')-$, $-CON(R')-$ oder $-COO-$ und R′ für Wasserstoff, unsubstituiertes oder durch eine Cyanogruppe substituiertes Alkyl mit 1 bis 4 Kohlenstoffatomen oder zusammen mit $R_4$ für einen Piperazinring. Die Benzol- und die heterocyclischen Ringe sind vorzugsweise mit Wasserstoff, Chlor, Alkyl mit 1 bis 4 Kohlenstoffatomen oder Alkoxy mit 1 bis 4 Kohlenstoffatomen substituiert, der Benzimidazolring ausserdem noch mit Alkylsulfonyl mit 1 bis 4 Kohlenstoffatomen, Phenylsulfonyl, Cyano, Trifluormethyl, Phenoxysulfonyl, Alkoxycarbonyl mit insgesamt 2 bis 5 Kohlenstoffatomen, Carboxy, $-CONZ'_1Z'_2$ oder $-SO_2NZ'_1Z_2$, worin $Z'_1$ für Wasserstoff, Alkyl mit 1 bis 4 C-Atomen, Alkenyl mit 3 oder 4 C-Atomen, Hydroxyalkyl mit 2 bis 4 C-Atomen, Alkoxyalkyl mit insgesamt 3 bis 6 C-Atomen, Cyclohexyl oder Benzyl und $Z_2'$ für Wasserstoff, Alkyl mit 1 bis 4 C-Atomen stehen oder $Z_1'$ und $Z_2'$ zusammen mit dem Stickstoffatom, an das sie gebunden sind, auch einen Morpholinring bilden können.

Von besonderer Bedeutung sind kationische Aufheller der Formel

(10) 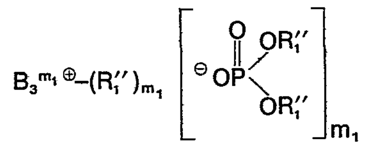

worin $m_1$ 1 oder 2 und $R_1''$ Alkyl mit 1 bis 3 C-Atomen und $B_3$ einen Aufhellerrest der Formel

(11) 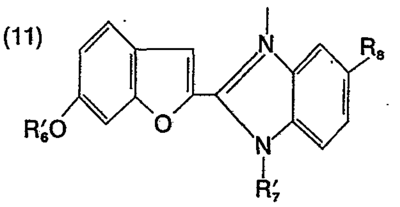,

(12) 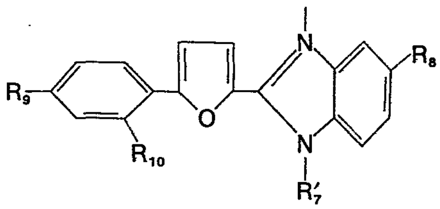,

(13). 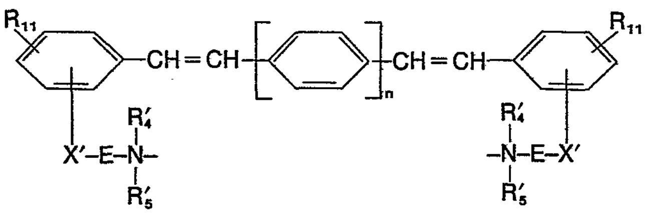

oder

(14) 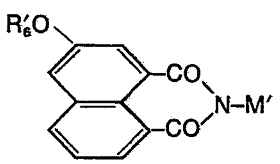

bedeuten, in welchen Reste

$R_6'$ Alkyl mit 1 bis 4 C-Atomen,

$R_7'$ Alkyl mit 1 bis 4 C-Atomen, Benzyl, Phenyl, Alkenyl mit 3 bis 4 C-Atomen, Cyanoalkyl mit 2 oder 3 C-Atomen, Alkoxycarbonylmethyl mit 1 bis 3 C-Atomen im Alkoxyteil,

$R_8$ Wasserstoff, Methyl, Chlor, Alkylsulfonyl mit 1 bis 4 C-Atomen, Phenylsulfonyl, Cyano, Trifluormethyl, Phenoxysulfonyl, Alkoxycarbonyl mit insgesamt 2 bis 5 C-Atomen, Carboxy, $-CONZ'_1Z'_2$ oder $-SO_2NZ'_1Z'_2$, worin $Z'_1$ für Wasserstoff, Alkyl mit 1 bis 4 C-Atomen, Alkenyl mit 3 oder 4 C-Atomen, Hydroxyalkyl mit 2 bis 4 C-Atomen, Alkoxyalkyl mit insgesamt 3 bis 6 C-Atomen, oder Benzyl und $Z'_2$ für Wasserstoff, Alkyl mit 1 bis 4 C-Atomen stehen oder $Z'_1$ und $Z'_2$ zusammen mit dem Stickstoffatom, an das sie gebunden sind, auch einen Morpholinring bilden können,

$R_9$ und $R_{10}$, unabhängig voneinander Wasserstoff, Chlor oder Methyl,

R′ Wasserstoff, unsubstituiertes oder durch eine Cyanogruppe substituiertes Alkyl mit 1 bis 4 C-Atomen oder zusammen mit $R_4'$ einen Piperazinrest,

X′ Sauerstoff, Schwefel, die direkte Bindung, $-SO_2NR'-$, $-CONR'-$ oder $-COO-$,

E unsubstituiertes oder durch eine OH-Gruppe substituiertes Alkylen mit 1 bis 4 C-Atomen,

$R'_4$ Alkyl mit 1 bis 4 C-Atomen, zusammen mit R′ einen Piperazinrest oder zusammen mit $R'_5$ und dem Stickstoffatom, an das sie gebunden sind, einen Pyrrolidin-, Piperidin-, Hexamethylenimin- oder Morpholinrest,

$R'_5$ Alkyl mit 1 bis 4 C-Atomen oder zusammen mit $R'_4$ und dem Stickstoffatom, an das sie gebunden sind, einen Pyrrolidin-, Piperidin-, Hexamethylenimin- oder Morpholinrest,

$R_{11}$ Wasserstoff, Chlor, Alkyl mit 1 bis 4 C-Atomen oder Alkoxy mit 1 bis 4 C-Atomen,

n die Zahl 1 oder 2 und

M′ -Alkylen $\overset{|}{N}(R'_4R'_5)$ oder den Rest

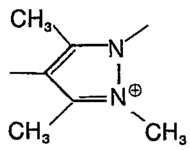

bedeuten.

Von besonderem Interesse sind

a) kationische Aufheller der Formel

(15) 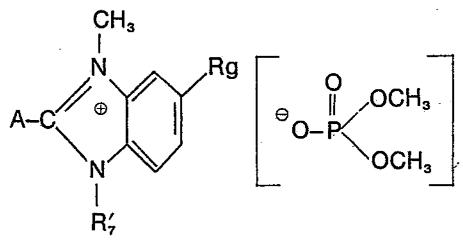

worin A einen Rest der Formel

(16) 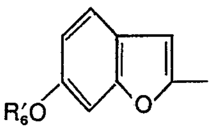 oder (17) 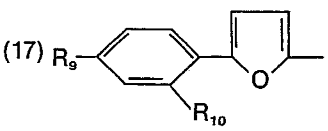

wobei $R'_6$ für Alkyl mit 1 bis 4 C-Atomen und $R_9$ und $R_{10}$ unabhängig voneinander für Wasserstoff, Chlor oder Methyl stehen, $R'_7$ Alkyl mit 1 bis 4 C-Atomen, Benzyl, Phenyl, Alkenyl mit 3 oder 4 C-Atomen, Cyanoalkyl mit 2 oder 3 C-Atomen, Alkoxycarbonylmethyl mit 1 bis 3 C-Atomen im Alkoxyteil und $R_8$ Wasserstoff, Methyl, Chlor, Alkylsulfonyl mit 1 bis 4 C-Atomen, Phenylsulfonyl, Cyano, Trifluormethyl, Phenoxysulfonyl, Alkoxycarbonyl mit insgesamt 2 bis 5 C-Atomen, Carboxy, $-CONZ'_1Z'_2$ oder $-SO_2NZ'_1Z'_2$, worin $Z'_1$ für Wasserstoff, Alkyl mit 1 bis 4 C-Atomen, Alkenyl mit 3 oder 4 C-Atomen, Hydroxyalkyl mit 2 bis 4 C-Atomen, Alkoxyalkyl mit insgesamt 3 bis 6 C-Atomen oder Benzyl und $Z'_2$ für Wasserstoff, Alkyl mit 1 bis 4 C-Atomen stehen oder $Z'_1$ und $Z'_2$ zusammen mit dem Stickstoffatom, an das sie gebunden sind, auch einen Morpholinring bilden können, bedeuten, sowie

b) kationische Aufheller der Formel

(18) 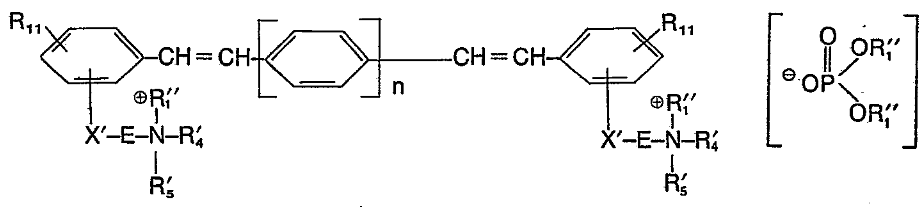

worin $R''_1$ Alkyl mit 1 bis 3 C-Atomen, vorzugsweise Methyl, X' Sauerstoff, Schwefel, die direkte Bindung, –COO–, –CON(R'')– oder $-SO_2N(R'')-$, wobei X' vorzugsweise in 2- oder 3-Stellung ist, R'' Wasserstoff, Alkyl mit 1 bis 4 C-Atomen oder zusammen mit $R'_4$ einen Piperazinrest, E unsubstituiertes oder durch eine OH-Gruppe substituiertes Alkylen mit 1 bis 4 C-Atomen, $R'_4$ Alkyl mit 1 bis 4 C-Atomen, zusammen mit R'' einen Piperazinrest oder zusammen mit $R'_5$ und dem Stickstoffatom, an das sie gebunden sind, einen Pyrrolidin-, Piperidin-, Hexamethylenimin- oder Morpholinrest, $R'_5$ Alkyl mit 1 bis 4 C-Atomen oder zusammen mit $R'_4$ und dem Stickstoffatom, an das sie gebunden sind, einen Pyrrolidin-, Piperidin-, Hexamethylenimin- oder Morpholinrest, $R_{11}$ Wasserstoff, Chlor, Alkyl mit 1 bis 4 C-Atomen oder Alkoxy mit 1 bis 4 C-Atomen und n die Zahl 1 oder 2 bedeuten.

Ganz besondere Bedeutung haben

c) kationische Aufheller der Formel

(19) 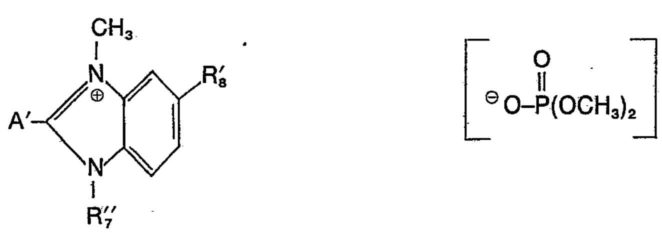

worin A' einen Rest der Formel

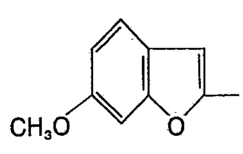 oder 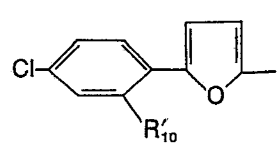

$R''_7$ Alkyl mit 1 bis 4 C-Atomen, Benzyl, Phenyl oder Cyanoalkyl mit 2 bis 3 Kohlenstoffatomen, $R'_8$ Wasserstoff, Alkylsulfonyl mit 1 bis 4 Kohlenstoffatomen, Phenylsulfonyl, Phenoxysulfonyl, Sulfamoyl, Alkylsulfamoyl mit 1 bis 4 Kohlenstoffatomen, Alkoxycarbonyl mit 2 bis 4 Kohlenstoffatomen, Cyano oder Chlor und $R'_{10}$ Wasserstoff, Chlor oder Methyl bedeuten, sowie

d) kationische Aufheller der Formel

(20) 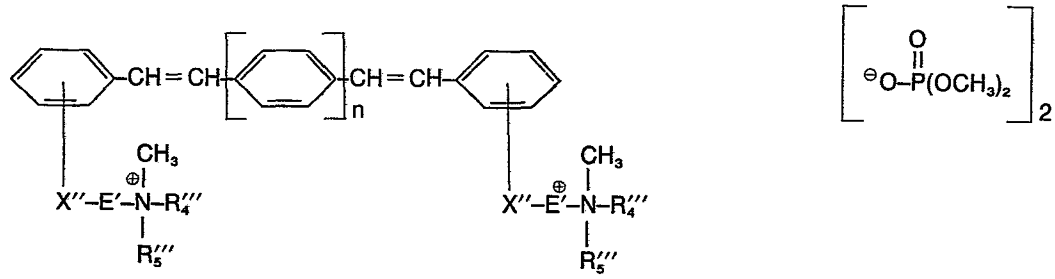

worin X″ Sauerstoff, die direkte Bindung, $-SO_2NH-$ oder $-CONH-$ in 2- oder 3-Stellung, E′ Alkylen mit 1 bis 3 C-Atomen, $R'''_4$ und $R'''_5$ Alkyl mit 1 oder 2 C-Atomen oder $R'''_4$ und $R'''_5$ zusammen mit dem Stickstoffatom, an das sie gebunden sind, einen Pyrrolidin-, Piperidin- oder Morpholinrest und n die Zahl 1 oder 2 bedeuten.

Die Herstellung von kationischen Aufhellern der Formel (1) erfolgt in an sich bekannter Weise durch Quaternierung eines Aufhellers B mit mindestens stöchiometrischen Mengen m eines Phosphates der Formel

(21) 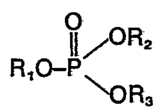

worin $R_1$, $R_2$ und $R_3$ die oben angegebene Bedeutung haben, bei Temperaturen zwischen 60 und 200 °C, gegebenenfalls in einem inerten Reaktionsmedium.

Als Phosphate der Formel (21) kommen vorzugsweise in Betracht: Trimethylphosphat, Triäthylphosphat, Triisopropylphosphat, Tri-n-propylphosphat, Dibutylmethylphosphat, Tribenzylphosphat, Diisopropylmethylphosphat, Triallylphosphat, Tributylphosphat, Tris-(2-chloräthyl)-phosphat und Tris-(methoxyäthyl)-phosphat. Besonders bevorzugt ist Trimethylphosphat. Mit diesen Trialkylphosphaten können auch Basen von kationischen Farbstoffen quaterniert werden.

Als Reaktionsmedia in denen die Quaternierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Dichloräthan, Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Äther wie Methanol, Äthanol, Butanol, Dibutyläther, Äthylenglykol, Äthylenglykolmonomethyläther, Äthylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonsäure-ester oder -nitrile wie Essigester, Essigsäurebutylester, Acetonitril oder Methoxypropionitril. Dabei wird z.B. bei Temperaturen von 60 bis 200 °C, vorzugsweise 80 bis 170 °C gearbeitet. Zuweilen ist es auch vorteilhaft, Wasser als Lösungsmittel zu verwenden.

Nach einem besonders vorteilhaften Verfahren arbeitet man in überschüssigem Alkylierungsmittel bei 60 bis 200 °C, vorzugsweise 100 bis 170 °C, wonach sich das Reaktionsprodukt oft entweder beim Abkühlen oder durch Zugabe eines geeigneten Lösungsmittels, z.B. eines der vorangehend genannten Lösungsmittel, ausscheidet.

Da die erhaltenen kationischen Aufheller besonders gut wasserlöslich sind, kann man ein Reaktionsgemisch, das noch wasserlösliches, überschüssiges Phosphat der Formel (21) enthält, auch ohne Isolierung des Aufhellers mit Wasser verdünnen, um derart zu einer gebrauchsfertigen, konzentrierten Lösung zu gelangen. Solche konzentrierte Lösungen sind ebenfalls Teil der vorliegenden Erfindung. Rein wässerige Lösungen erhält man durch Lösen der isolierten kationischen Aufheller in der gewünschten Menge Wasser.

Zur Stabilisierung der wässerigen Lösungen gegen eine allfällige Phosphorsäureester-hydrolyse kann diesen noch eine pH-Puffersubstanz zugegeben werden wie z.B. Natriumacetat oder Natriumlactat. Eine gute Pufferwirkung erhält man auch durch Neutralisation der zunächst meist sauer reagierenden, überschüssigen Phosphat enthaltenden wässerigen Lösungen mit Alkalihydroxiden (z.B. Natriumhydroxid), -carbonaten oder -bicarbonaten, wasserlöslichen basischen Aminen oder Ammoniak.

Die zur Quaternierung verwendeten Ausgangsstoffe der Formel (21) sind bekannt oder werden in Analogie zu an sich bekannten Verfahren hergestellt.

Die vorstehend definierten neuen Verbindungen zeigen in gelöstem oder feinverteiltem Zustande eine mehr oder weniger ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Die optisch aufzuhellenden organischen Materialien können den verschiedenartigsten Verarbeitungszuständen (Rohstoffe, Halbfabrikate oder Fertigfabrikate) angehören.

Den erfindungsgemäss anzuwendenden Verbindungen kommt u.a. Bedeutung für die Behandlung von textilen organischen Materialien, insbesondere textilen Geweben, zu.

In Abhängigkeit vom verwendeten Aufheller-Verbindungstyp kann es sich als vorteilhaft erweisen, in neutralem oder alkalischem oder saurem Bade zu verarbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäss vorliegender Erfindung können beispielsweise auch in folgenden Anwendungsformen eingesetzt werden.

a) Mischungen mit Farbstoffen (Nuancierung) oder Pigmenten (Farb- oder insbesondere z.B. Weisspigmenten) oder als Zusatz zu Färbebädern, Druck-, Ätz- oder Reservepasten. Ferner auch zur Nachbehandlung von Färbungen, Drukken oder Ätzdrucken,

b) in Mischungen mit Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie «wash-and-wear», «permanent-press», «noiron»), ferner Flammfest-, Weichgriff-, Schmutzablöse («anti-soiling»)- oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu sogenannten «master batches»,

f) als Zusätze zu den verschiedensten industriellen Produkten, um dieselben marktfähiger zu machen (z.B. Aspektverbesserung von Seifen, Waschmitteln, Pigmenten),

g) in Kombination mit anderen, optisch aufhellend wirkenden Substanzen,

h) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Szintillatoren, für verschiedene Zwecke photographischer Art, wie z.B. für elektrophotographische Reproduktion oder Supersensibilisierung.

Wird das Aufhellverfahren mit Textil-Behandlungs- oder Veredlungmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,005 und 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphaten oder Alkalimetallsilicaten.

Die erfindungsgemässen kationischen Aufheller sind geeignet zum optischen Aufhellen von organischen Materialien, besonders von Polyacrylnitril, Polyamid und Cellulose.

In den Beispielen sind Prozente immer Gewichtsprozente. Schmelz- und Siedepunkte sind, sofern nicht anders vermerkt, unkorrigiert und oft sehr unscharf.

Beispiel 1

7,1 g der Verbindung der Formel

(100)

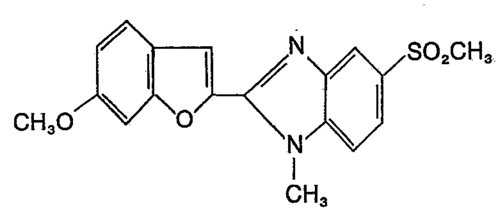

werden in 20 ml Trimethylphosphat unter Rühren und Überleiten eines schwachen Stickstoffstroms 60 Minuten auf 160 °C erhitzt. Nach dieser Zeit ist eine Probe klar löslich in Wasser. Während dem Abkühlen verdünnt man das Reaktionsgemisch mit 50 ml Essigsäureäthylester und nutscht das auskristallisierte Produkt bei Raumtemperatur ab.

Der an der Luft zerfliessliche Rückstand wird wiederholt mit Essigsäureäthylester gewaschen und sofort im Vakuum bei 100 °C getrocknet. Man erhält 10,8 g der Verbindung der Formel

(101)

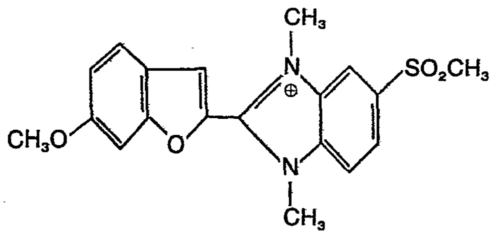

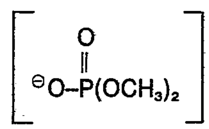

vom Smp. ~115 °C, die noch je 1 Mol Kristalltrimethylphosphat und Kristallwasser enthält.

Kristallisiert man dieses Produkt aus 1,2-Dichloräthan um oder versetzt man das bei der Herstellung entstandene, noch heisse Reaktionsgemisch mit Methyläthylketon anstatt mit Essigsäureäthylester, so erhält man ein Produkt vom Smp. 181 °C, das kein Kristall-dimethylphosphat mehr enthält. Die Löslichkeit dieses Produktes beträgt bei Raumtemperatur über 100 g in 100 ml Wasser, jene des entsprechenden Methosulfats 0,5 g.

Beispiel 2

7,1 g der Verbindung der Formel

(200)

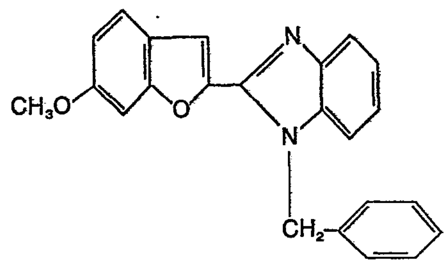

werden in 20 ml Trimethylphosphat unter Rühren und Überleiten eines schwachen Stickstoffstroms 60 Minuten auf 160 °C erhitzt. Nach dieser Zeit ist eine Probe klar löslich in Wasser. Nach dem Abkühlen auf Raumtemperatur erhält man die Verbindung der Formel

(201)

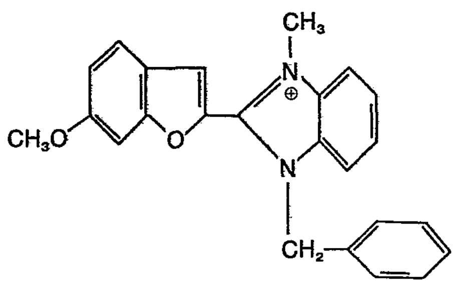

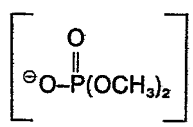

in überschüssigem Trimethylphosphat als dickflüssiges Öl, das ohne Ausscheidung beliebig mit Wasser verdünnt werden kann. Eine 19%-ige Lösung war nach mehrmonatigem Lagern unverändert im Aspekt. Nach Neutralisation einer Probe mit Natronlauge blieb der pH beim Lagern ebenfalls unverändert. Die Löslichkeit des entsprechenden Methosulfates in Wasser beträgt 0,5%.

In ähnlicher Weise wie in Beispiel 1 oder 2 beschrieben, werden die in Tabelle I aufgeführten Aufheller der Formel

(202)

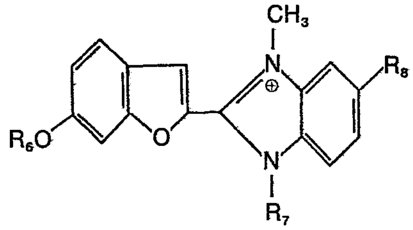

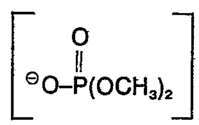

hergestellt:

Tabelle I

| Formel No. | $R_6$ | $R_7$ | $R_8$ | UV$\lambda_{max}$in Polyacrylnitril (nm) |
|---|---|---|---|---|
| 203 | $CH_3$ | $CH_3$ | $-SO_2NHCH_3$ | 365 |
| 204 | $CH_3$ | $CH_3$ | $-SO_2N(CH_3)_2$ | 365 |
| 205 | $CH_3$ | $CH_3$ | $-SO_2NH_2$ | 363 |
| 206 | $CH_3$ | $CH_3$ | 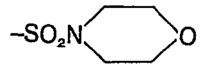 | 366 |
| 207 | $CH_3$ | $C_2H_5$ | $-SO_2NHC_2H_5$ | 362 |
| 208 | $CH_3$ | $CH_2C_6H_5$ | $-SO_2NH_2$ | 367 |
| 209 | $CH_3$ | $CH_2CH=CH_2$ | $-SO_2NHCH_2CH=CH_2$ | 366 |
| 210 | $CH_3$ | $n-C_4H_9$ | $-SO_2NH_2$ | 362 |
| 211 | $CH_3$ | $CH_3$ | $-SO_2NHCH_2CH_2OCH_3$ | 365 |
| 212 | $CH_3$ | $CH_2C_6H_5$ | $-SO_2NHCH_2C_6H_5$ | 367 |
| 213 | $CH_3$ | $C_2H_5$ | $-SO_2NH_2$ | 362 |
| 214 | $nC_4H_9$ | $CH_3$ | $-SO_2NHCH_3$ | 367 |
| 215 | $CH_3$ | $CH_3$ | $-COOH$ | 364 |
| 216 | $CH_3$ | $CH_3$ | $-COOC_2H_5$ | 364 |
| 217 | $CH_3$ | $C_2H_5$ | $-COOH$ | 362 |
| 218 | $CH_3$ | $CH_3$ | $-CONHCH_3$ | 360 |
| 219 | $CH_3$ | $CH_3$ | $-COOCH_3$ | 364 |
| 220 | $CH_3$ | $CH_2C_6H_5$ | $-CONH_2$ | 365 |
| 221 | $CH_3$ | $CH_3$ | $-CN$ | 370 |
| 222 | $CH_3$ | $CH_3$ | $-SO_2OC_6H_5$ | 370 |
| 223 | $CH_3$ | $CH_3$ | $-SO_2C_6H_5$ | 369 |
| 224 | $CH_3$ | $CH_3$ | $-CF_3$ | 360 |
| 225 | $CH_3$ | $CH_2C_6H_5$ | $-CF_3$ | 364 |
| 226 | $CH_3$ | $CH_2CH_2CN$ | H | 361 |
| 227 | $CH_3$ | $CH_2C_6H_5$ | $-Cl$ | 363 |
| 228 | $CH_3$ | $CH_3$ | $SO_2NHCH_2CH_2OH$ | 363 |
| 229 | $CH_3$ | $CH_2COOCH_3$ | H | 357 (in DMF) |
| 230 | $CH_3$ | $CH_2CN$ | H | 360 |
| 231 | $CH_3$ | $CH_2C_6H_5$ | $CH_3$ | 357 |

Beispiel 3

7,0 g der Verbindung der Formel

(300)

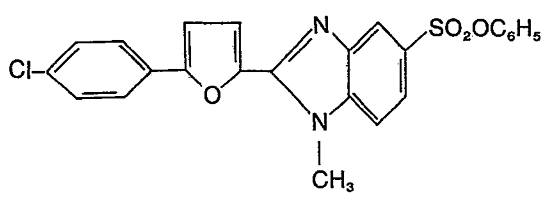

werden in 30 ml Trimethylphosphat unter Rühren und Überleiten von einem schwachen Stickstoffstrom 2 Stunden auf 160 °C erhitzt. Während dem Abkühlen verdünnt man das dick ausgefallene Produkt mit 30 ml Methyläthylketon und nutscht es bei Raumtemperatur ab. Der Rückstand wird wiederholt mit Methyläthylketon gewaschen und im Vakuum bei 100 °C getrocknet. Man erhält 7,0 g eines blassgelben, kristallinen Produktes der Formel

(301)

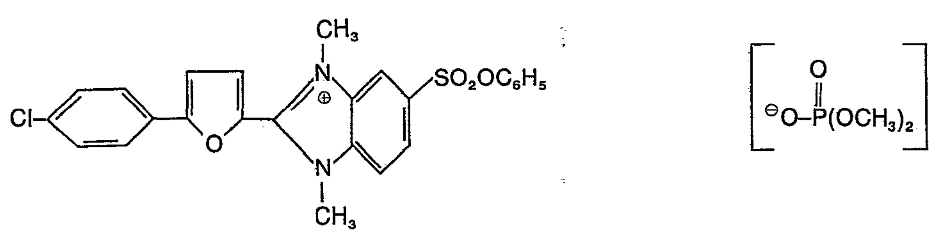

vom Smp. 246 °C. Die Löslichkeit dieses Produktes in Wasser beträgt bei 80 °C 10%, jene des entsprechenden Methosulfates 0,3%.

In analoger Weise erhält man die Verbindungen der Formeln

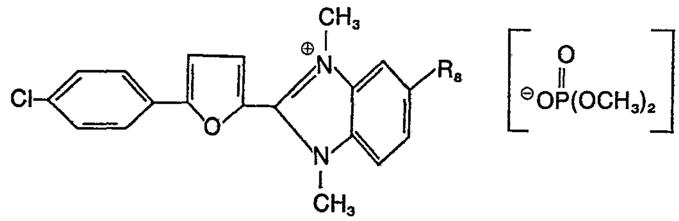

| No. | $R_8$ | Smp. | Löslichkeit in $H_2O$ bei Raumtemperatur |
|---|---|---|---|
| (302) | $-SO_2CH_3$ | 270°C | 17% |
| (303) | –CN | 220°C | 3,4% |

Die Löslichkeiten der entsprechenden Methosulfate in Wasser bei Raumtemperatur betragen je etwa 0,1%.

In der oben beschriebenen Weise können auch die in Tabelle II aufgeführten Aufheller der Formel

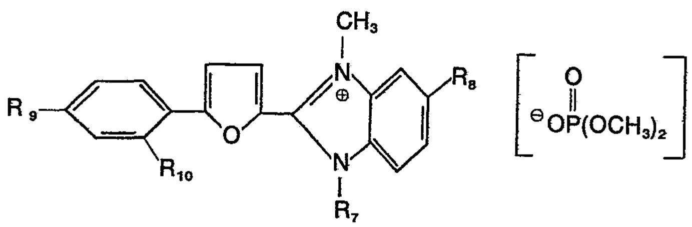

hergestellt werden.

Tabelle II

| Formel No. | $R_7$ | $R_{10}$ | $R_9$ | $R_8$ | $UV\lambda_{max}$ in Polyacrylnitril (nm) |
|---|---|---|---|---|---|
| 304 | $C_6H_5$ | Cl | Cl | H | 357 |
| 305 | $CH_3$ | H | $CH_3$ | $SO_2OC_6H_5$ | 357 |
| 306 | $CH_3$ | H | $COOC_2H_5$ | $SO_2CH_3$ | 363 |
| 307 | $CH_3$ | $CH_3$ | Cl | $SO_2CH_3$ | 360 |
| 308 | $CH_3$ | H | Cl | $SO_2C_6H_5$ | 366 |
| 309 | $CH_2C_6H_5$ | H | Cl | $SO_2CH_3$ | 364 |
| 310 | $CH_2CH_2CN$[1] | H | Cl | $SO_2CH_3$ | 367 |
| 311 | $CH_3$ | H | Cl | $SO_2C_2H_5$ | 364 |
| 312 | $C_2H_5$ | H | Cl | $SO_2CH_3$ | 361 |
| 313 | $CH_3$ | H | H | $SO_2CH_3$ | 362 |
| 314 | $CH_2CN$[1] | H | Cl | $SO_2CH_3$ | 372 |
| 315 | $CH_2C_6H_5$ | H | Cl | $SO_2N(CH_3)_2$ | 364 |
| 316 | $C_4H_9$ | H | Cl | $SO_2NH_2$ | 361 |
| 317 | $CH_2C_6H_5$ | H | Cl | $SO_2NHCH_2C_6H_5$ | 365 |
| 318 | $CH_2CH=CH_2$ | H | Cl | $SO_2NHCH_2CH=CH_2$ | 362 |
| 319 | $CH_3$ | H | Cl | $CONHCH_3$ | 362 |
| 320 | $CH_3$ | H | Cl | $COOCH_3$ | 363 |
| 321 | $CH_3$ | H | Cl | COOH | 363 |
| 322 | $CH_2C_6H_5$ | H | Cl | $CONH_2$ | 363 |

[1] Isomerenmischung ($R_7$ und $CH_3$ vertauscht)

Beispiel 4

5,6 g der Verbindung der Formel

(400) 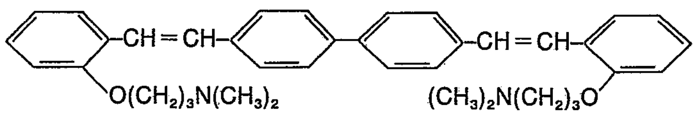

werden in 30 ml Trimethylphosphat unter Rühren während 60 Minuten auf 100 °C erhitzt, wobei das Reaktionsprodukt auskristallisiert. Nach dieser Zeit ist eine Probe davon klar löslich in Wasser. Man verdünnt mit 30 ml Methyläthylketon, lässt abkühlen, saugt ab und wäscht den Rückstand wiederholt mit Methyläthylketon. Nach dem Trocknen im Vakuum bei 100 °C erhält man 7,2 g der Verbindung der Formel

(401) 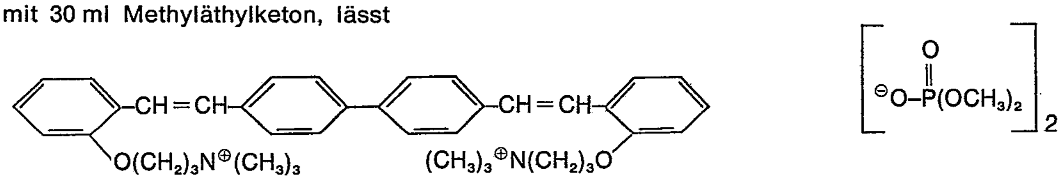

in annähernd farblosen Kristallen vom Smp. 270 °C (Zers.).

Dieses Produkt ist in Wasser bei Raumtemperatur in einer Konzentration von etwa 40% klar löslich verglichen mit 11% für das entsprechende Methosulfat.

In analoger Weise erhält man die Verbindung der Formel

(402) 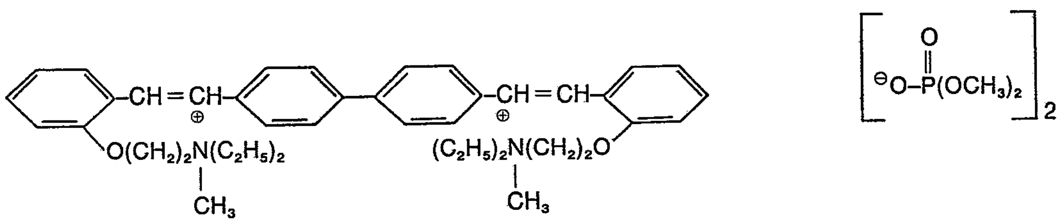

vom Smp. 190 °C, die etwa 3½ Mol Kristallwasser enthält. Die Löslichkeit dieses Produktes in Wasser beträgt mindestens 50%, diejenige des entsprechenden Metholsulfates 16%.

Erhitzt man die Verbindung der Formel (400) anstatt mit Trimethylphosphat mit Triäthylphosphat bei 150 °C und arbeitet ansonsten wie beschrieben, so erhält man 6,5 g der Verbindung der Formel

(403) 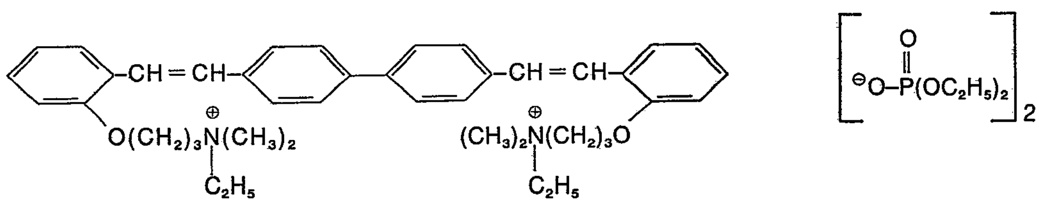

welche aus 1,2-Dichloräthan umkristallisiert werden kann und 1½ Mol Kristallwasser enthält (Smp. ca. 153 °C). die Löslichkeit dieses Produktes in Wasser beträgt etwa 40%.

In ähnlicher Weise wie in den vorangehenden Beispielen beschrieben, werden die in Tabelle III aufgeführten Aufheller der Formel

(404)

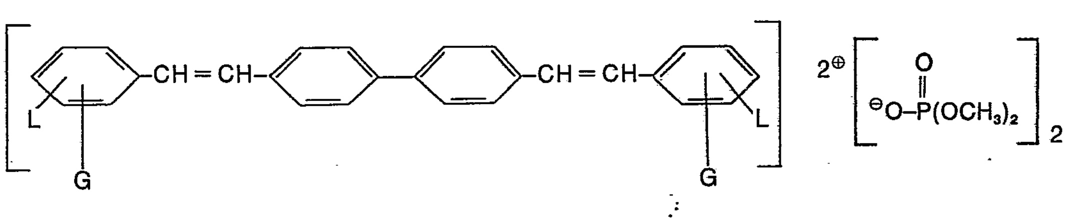

hergestellt.

Tabelle III

| Formel Nr. | G | L | $\lambda_{max}$ in Dimethylformamid (nm) |
|---|---|---|---|
| 405 | $2-O(CH_2)_2-N(CH_3)_3$ | H | 362 |
| 406 | $2-SO_2NH(CH_2)_3-N(CH_3)_3$ | H | 355 |
| 407 | $2-SO_2NH(CH_2)_2-N(CH_3)_3$ | H | 357 |
| 408 | $2-SO_2NH(CH_2)_3-NCH_3(C_2H_5)_2$ | H | 351 |
| 409 | 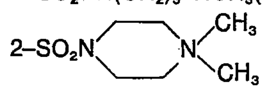 | H | 357 |
| 410 | $3-SO_2NH(CH_2)_3-N(CH_3)_3$ | 4–Cl | 362 |
| 411 | $2-CONH(CH_2)_3-N(CH_3)_3$ | H | 351 |
| 412 | 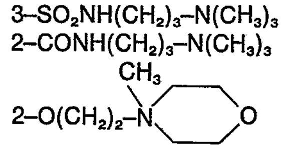 | H | 361 |
| 413 | 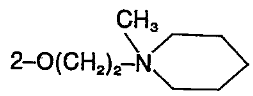 | H | 360 |
| 414 | 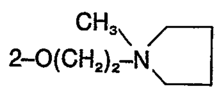 | H | 361 |
| 415 | $2-OCHCH_2-N(CH_3)_3$<br>\|<br>$CH_3$<br>Mischung | H | 361 |
| 416 | $2-OCH_2-CH-N(CH_3)_3$<br>\|<br>$CH_3$ | H | |
| 417 | $3-O(CH_2)_2-N(C_2H_5)_2$<br>\|<br>$CH_3$ | H | 356 |
| 418 | $2-OCH_2CHCH_2-N(CH_3)_3$<br>\|<br>OH | H | 362 |
| 419 | $2-S(CH_2)_2-N(CH_3)_3$ | H | 358 |
| 420 | $2-O(CH_2)_3-N(CH_3)_3$ | $3-OCH_3$ | 356 |
| 421 | $2-O(CH_2)_3-N(CH_3)_3$ | 5–Cl | 367 |
| 422 | $2-O(CH_2)_3-N(CH_3)_3$ | $5-CH_3$ | 366 |
| 423 | $2-CH_2N(CH_3)_3$ | H | 352 |
| 424 | $4-O-(CH_2)_2-N(C_2H_5)_2$<br>\|<br>$CH_3$ | H | 362 |
| 425 | $2-COO-(CH_2)_2N(CH_3)_3$ | H | 356 |

Beispiel 5

5,1 g der Verbindung der Formel

(500) 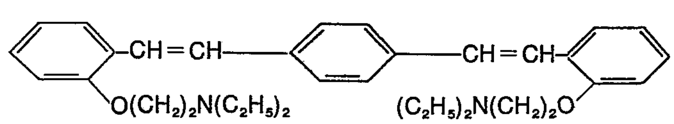

werden in 10 ml Trimethylphosphat unter Rühren während 60 Minuten auf 100 °C erhitzt. Nach dieser Zeit ist eine Probe des Reaktionsgemisches klar löslich in Wasser. Man verdünnt mit 30 ml Methyläthylketon, lässt abkühlen, nutscht das auskristallisierte Produkt ab und wäscht es wiederholt mit Methyläthylketon. Nach dem Trocknen im Vakuum bei 100 °C erhält man 5,1 g der Verbindung der Formel

(501) 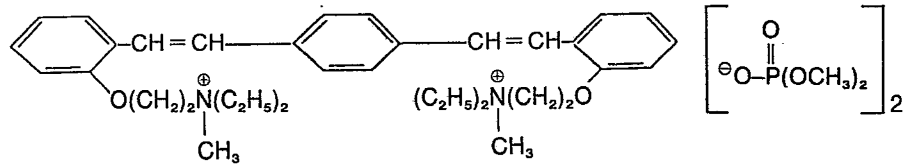

in Form von blassgelben Kristallen vom Smp. 187 °C. Die Löslichkeit dieses Produktes beträgt bei Raumtemperatur in Wasser über 50%.

In analoger Weise erhält man die Verbindung der Formel

(502) 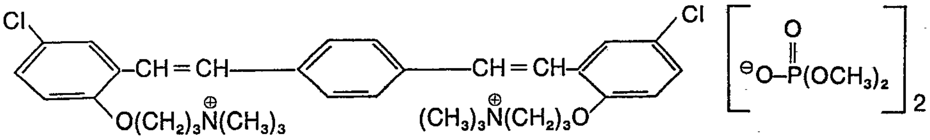

vom Smp. 250 °C. Die Löslichkeit dieses Produktes in Wasser beträgt bei Raumtemperatur über 50%, verglichen mit 16% für das entsprechende Methosulfat.

In ähnlicher Weise wie in den vorangehenden Beispielen beschrieben, werden die in Tabelle IV aufgeführten Aufheller der Formel

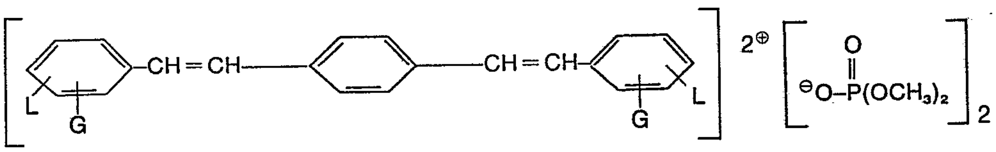

hergestellt.

Tabelle IV

| Formel No. | G | L | $\lambda_{max}$ in Dimethylformamid (nm) |
|---|---|---|---|
| 503 | $2–O–(CH_2)_3–N(CH_3)_3$ | H | 367 |
| 504 | $2–O–(CH_2)_2–N(CH_3)_3$ | H | 367 |
| 505 | 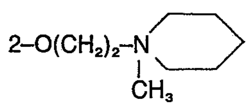 | H | 367 |
| 506 | 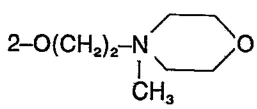 | H | 364 |
| 507 | 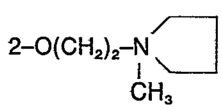 | H | 367 |
| 508 | $3–O(CH_2)_3–N(C_2H_5)_2$<br>\|<br>$CH_3$ | H | 358 |
| 509 | $4–O(CH_2)_2–N(C_2H_5)_2$<br>\|<br>$CH_3$ | H | 367 |
| 510 | $2–O–(CH_2)_3N(CH_3)_3$ | $3–OCH_3$ | 360 |
| 511 | $2–O(CH_2)_3–N(CH_3)_3$ | $5–CH_3$ | 372 |
| 512 | $3–SO_2NH(CH_2)_3–N(CH_3)_3$ | 4–Cl | 366 |
| 513 | $2–SO_2NH(CH_2)_3–N(CH_3)_3$ | H | 353 |
| 514 | $3–CONH(CH_2)_3–N(CH_3)_3$ | $6–CH_3$ | 367 |
| 515 | $2–CH_2N(CH_3)_3$ | H | 352 |

Beispiel 6

6,25 g der Verbindung der Formel

(600) 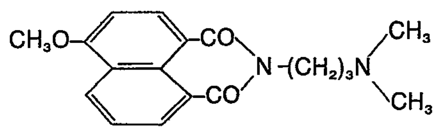

werden in 5,6 g Trimethylphosphat unter Rühren 5 Minuten auf 80 °C erwärmt. Nach dieser Zeit ist eine Probe klar löslich in Wasser und das Reaktionsprodukt fällt dick aus. Man verdünnt mit 50 ml Methyläthylketon, lässt abkühlen und filtriert. Der an der Luft zerfliessliche Rückstand wird wiederholt mit Methyläthylketon gewaschen und sofort im Vakuum bei 100 °C getrocknet. Man erhält 8,8 g der Verbindung der Formel

(601) 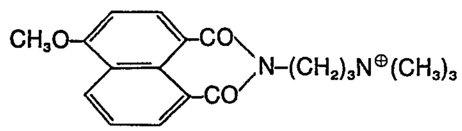 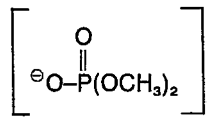

vom Smp. 188 °C. Die Löslichkeit dieses Produktes in Wasser beträgt bei Raumtemperatur über 60% verglichen mit 44% mit dem entsprechenden Salz der Phosphorsäure.

In ähnlicher Weise wie in den vorangehenden Beispielen erwähnt, erhält man die Verbindung der Formel

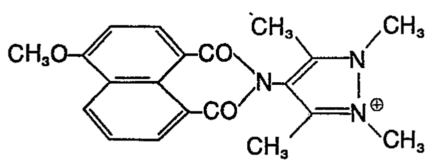 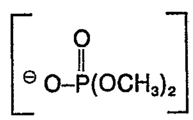

welche ohne Isolierung nach Verdünnen mit Wasser verwendet wird (UV$\lambda_{max}$ 365 nm in DMF).

Beispiel 7

Ein Polyacrylnitril-Gewebe (Orlon®75) wird auf einem Färbeapparat bei einem Flottenverhältnis von 1 : 20 mit einem wässrigen Bad, das

0,1% des Aufhellers der Formel (101), (201), (203), (301), (302), (303), (402), (403), (406), (501) oder (512) bezogen auf das Warengewicht,

1 g/l eines Anlagerungsproduktes von 35 Mol Äthylenoxid an 1 Mol Stearylalkohol und

1,5 ml/l Ameisensäure 85%

enthält, behandelt.

Die Applikation erfolgt gemäss folgendem Temperaturprogramm:

40–97 °C/30 Minuten

97 °C/30 Minuten

97–40 °C/15 Minuten.

Anschliessend wird das Polyacrylnitril-Gewebe während 30 Sekunden in fliessendem enthärtetem Wasser gespült und bei 70 °C im Trockenschrank getrocknet. Das so behandelte Gewebe weist einen guten Aufhelleffekt auf.

Beispiel 8

Ein modifiziertes Polyacrylnitrilgewebe (Courtelle®) wird auf einem Färbeapparat bei einem Flottenverhältnis von 1 : 20 mit einem wässerigen Bad, das

0,1% des Aufhellers der Formel (101), (201), (203), (301) bis (303), (401), (402), (403), (406), (501) oder (512) bezogen auf das Warengewicht,

1 g/l Oxalsäure

0,25 g/l eines Polyphosphates als Komplexbildner und

0,125 g/l Natriummetabisulfit

enthält, behandelt. Die Applikation erfolgt gemäss folgendem Temperaturprogramm:

40–97 °C/30 Minuten

97 °C/30 Minuten

97–40 °C/15 Minuten.

Anschliessend wird das Polyacrylnitril-Gewebe während 30 Sekunden in fliessendem enthärtetem Wasser gespült und bei 70 °C im Trockenschrank getrocknet. Das so behandelte Gewebe weist einen guten Aufhelleffekt auf.

Beispiel 9

Frisch ausgesponnenes, verstrecktes Polyacrylnitril-Nasskabel (entsprechend 3,0 Trockengewicht) wird noch feucht bei 45 °C für 4 Sekunden in 100 ml einer wässrigen Flotte getaucht, die 0,0005% eines Aufhellers der Formel (101), (301), (401), (406) oder (501) enthält und mit konzentrierter Oxalsäurelösung auf pH 4 eingestellt worden ist. Anschliessend spült man das Nasskabel kurz mit Wasser und trocknet bei 90 bis 100 °C. Man erhält auf diese Weise eine gut aufgehellte Polyacrylnitrilfaser.

Die Ausfärbung kann z. B. auch bei pH 6 (eingestellt durch Zugabe von Natriumacetat) erfolgen. Erhöhte Temperatur der Färbeflotte, z. B. auf 40 °C, erhöht die Ausziehgeschwindigkeit.

Höhere Weisseffekte werden durch Erhöhen der Aufhellerkonzentration, z. B. auf 0,005%, erzielt.

Beispiel 10

Ein gebleichtes Baumwoll-Gewebe wird auf einem Färbeapparat bei einem Flottenverhältnis von 1 : 20 mit einem wässrigen Bad behandelt, das 0,1% des Aufhellers der Formel (401), (402), (403), (406), (501) oder (512), bezogen auf das Gewicht der Baumwolle, und 5 g/l Natriumsulfat enthält.

Die Applikation erfolgt gemäss folgendem Temperaturprogramm:

20–50 °C/15 Minuten
50 °C/15 Minuten

Anschliessend wird das Baumwoll-Gewebe während 20 Sekunden in fliessendem enthärtetem Wasser gespült und bei 70 °C im Trockenschrank getrocknet. Das so behandelte Baumwoll-Gewebe weist einen guten Aufhelleffekt auf.

Beispiel 11

Man foulardiert bei Raumtemperatur ein gebleichtes Baumwoll-Gewebe mit einer wässrigen Flotte, die 1 g/l des Aufhellers der Formel (401), (402), (403), (406) oder (501) enthält. Der Abquetscheffekt beträgt 75%.

Anschliessend wird während 30 Sekunden bei 130 °C auf einem Thermofixiergerät getrocknet.

Das so behandelte Baumwollgewebe weist einen guten Aufhelleffekt auf.

Beispiel 12

Ein konzentriertes flüssiges Waschmittel wird durch Mischen folgender Komponenten hergestellt:

| | Gew.-% |
|---|---|
| Äthoxylierte Alkohole ($C_{12}$–$C_{13}$ Alkohol mit 6,5 Mol Äthylenoxid) | 60,0 |
| 1-Methyl-1-oleylamidoäthyl-2-oleyl-imidazoliniummethosulfat | 26,7 |
| Verbindung der Formel (401), (402), (501) oder (512) | 0,3 |
| Wasser | 12,0 |
| Übliche Zusätze | 1,0 |

2 kg gebleichtes Baumwollgewebe werden während 10 Minuten bei 50 °C in 60 Litern Wasser von 100 ppm Härte gewaschen, das 50 bis 60 g des obigen Waschmittels enthalten. Nach dem Spülen und Trocknen weist das Gewebe einen starken Aufhelleffekt und einen weichen Griff auf.

Ähnliche Resultate werden erhalten, wenn man anstelle der oben angegebenen Imidazolinium-Verbindung nicht-gehärtetes Di-talg-dimethylammoniumchlorid verwendet.

Beispiel 13

Ein gebleichtes Baumwoll-Gewebe wird im Flottenverhältnis 1 : 20 während 15 Minuten in einer 40 °C warmen, wässrigen Flotte gewaschen, die pro Liter

0,5 g eines Anlagerungsproduktes von 10 Mol Äthylenoxid an einem Mol Stearylalkohol und

0,01 g des Aufhellers der Formel (401), (402), (403), (405) oder (501) enthält.

Anschliessend wird das Baumwoll-Gewebe während 20 Sekunden in fliessendem Trinkwasser gespült und bei 70 °C im Trockenschrank getrocknet. Das so behandelte Baumwoll-Gewebe weist einen guten Aufhelleffekt auf.

**Patentansprüche**

1. Kationische Aufheller der Formel

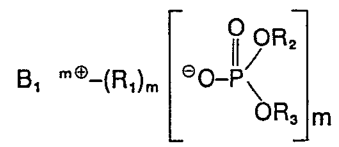

worin $B_1$ einen Aufheller aus der Gruppe der 2-Furanyl-benzimidazole, 2-Azolyl-benzimidazole, 2-Stilbenyl-benzimidazole, basischen 1,2-Bis-(azolyl)-äthylene, 2,5-Bis-(benzimidazolyl)-furane, basischen 4,4′-Bis-(azolyl)-stilbene, basischen 2-Phenyl-5-azolyl-thiophene, basischen 4,4′-Distyryl-biphenyle, basischen 1,4-Distyryl-benzole, basischen 3,7-disubstituierten Cumarine, basischen Naphthalimide, basischen 4,4′-Triazinylamino-stilbene, basischen 2-Stilben-4-yl-naphthotriazole und basischen Triazolyl- oder Pyrazolyl-stilbene, m die Anzahl basischer Aminogruppen und $R_1$, $R_2$ und $R_3$ unsubstituiertes oder nicht-chromophor substituiertes Alkyl mit 1 bis 4 C-Atomen oder unsubstituiertes oder nicht-chromophor substituiertes Alkenyl mit 2 bis 4 C-Atomen bedeuten.

2. Kationische Aufheller gemäss Anspruch 1 der Formel

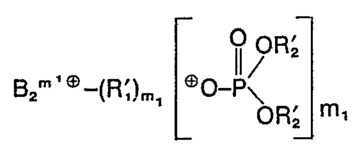

worin $m_1$ 1 oder 2 bedeutet und $R'_1$ und $R'_2$ Alkyl mit 1 bis 4 C-Atomen oder Benzyl und $B_2$ einen Aufheller der Formel

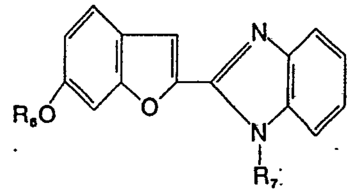 , 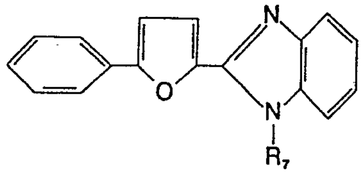 ,

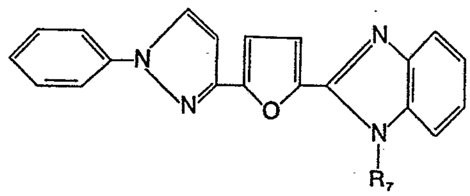

,

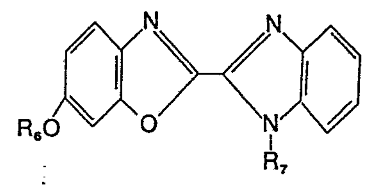

,

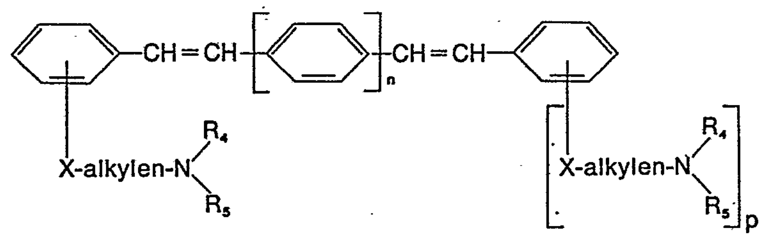

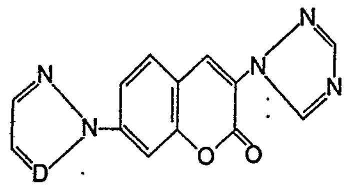

oder

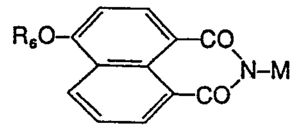

worin M Alkylen-N($R_4$)($R_5$), Pyrazolyl oder Pyridinyl, $R_4$ und $R_5$ unabhängig voneinander unsubstituiertes oder nicht-chromophor substituiertes Alkyl mit 1 bis 6 C-Atomen, unsubstituiertes oder nicht-chromophor substituiertes Alkenyl mit 2 bis 6 C-Atomen oder $R_4$ und $R_5$ zusammen mit dem N-Atom auch einen 5- bis 7-gliedrigen heterocyclischen Ring, $R_6$ und $R_7$ unabhängig voneinander unsubstituiertes oder nicht-chromophor substituiertes Alkyl mit 1 bis 6 C-Atomen, unsubstituiertes oder nicht-chromophor substituiertes Alkenyl mit 2 bis 6 C-Atomen oder Phenyl, D Stickstoff oder die CH-Gruppe, X ein nicht-chromophores Brükkenglied oder die direkte Bindung, n die Zahl 1 oder 2 und p die Zahl 0 oder 1 bedeuten, wobei die Benzol- und die heterocyclischen Ringe auch nicht-chromophor substituiert sein können.

3. Kationische Aufheller gemäss Anspruch 2 der Formel

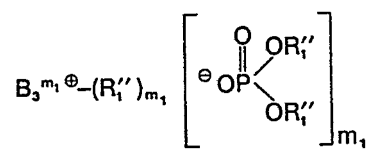

worin $m_1$ 1 oder 2 und $R''_1$ Alkyl mit 1 bis 3 C-Atomen und $B_3$ einen Aufhellerrest der Formel

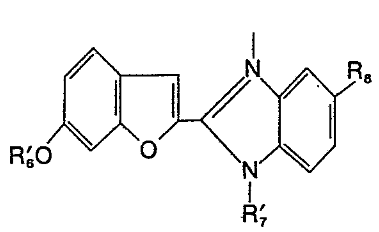

,

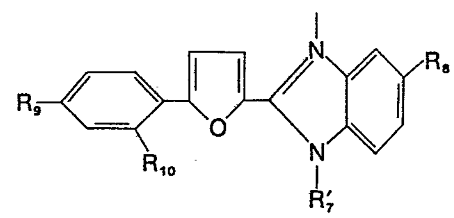

,

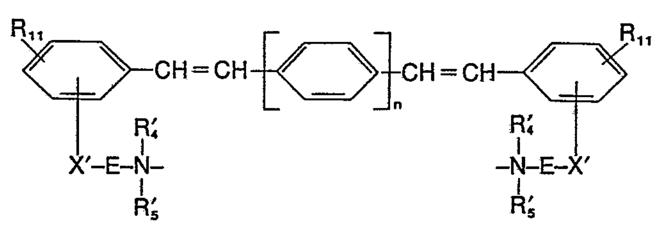

oder

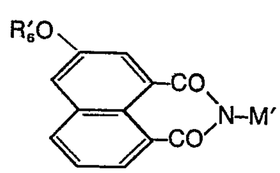

bedeuten, in welchen Reste

$R'_6$ Alkyl mit 1 bis 4 C-Atomen,

$R'_7$ Alkyl mit 1 bis 4 C-Atomen, Benzyl, Phenyl, Alkenyl mit 3 bis 4 C-Atomen, Cyanoalkyl mit 2 oder 3 C-Atomen, Alkoxycarbonylmethyl mit 1 bis 3 C-Atomen im Alkoxyteil,

$R_8$ Wasserstoff, Methyl, Chlor, Alkylsulfonyl mit 1 bis 4 C-Atomen, Phenylsulfonyl, Cyano, Trifluormethyl, Phenoxysulfonyl, Alkoxycarbonyl mit insgesamt 2 bis 5 C-Atomen, Carboxy, $-CONZ'_1Z'_2$ oder $-SO_2NZ'_1Z'_2$, worin $Z'_1$ für Wasserstoff, Alkyl mit 1 bis 4 C-Atomen, Alkenyl mit 3 oder 4 C-Atomen, Hydroxyalkyl mit 2 bis 4 C-Atomen, Alkoxyalkyl mit insgesamt 3 bis 6 C-Atomen, oder Benzyl und $Z'_2$ für Wasserstoff, Alkyl mit 1 bis 4 C-Atomen stehen oder $Z'_1$ und $Z'_2$ zusammen mit dem Stickstoffatom auch einen Morpholinring bilden können,

$R_9$ und $R_{10}$ unabhängig voneinander Wasserstoff, Chlor oder Methyl,

R' Wasserstoff, unsubstituiertes oder durch eine Cyanogruppe substituiertes Alkyl mit 1 bis 4 C-Atomen oder zusammen mit $R'_4$ einen Piperazinrest,

X' Sauerstoff, Schwefel, die direkte Bindung, $-SO_2NR'-$, $-CONR'-$ oder $-COO-$,

E unsubstituiertes oder durch eine OH-Gruppe substituiertes Alkylen mit 1 bis 4 C-Atomen,

$R'_4$ Alkyl mit 1 bis 4 C-Atomen, zusammen mit R' einen Piperazinrest oder zusammen mit $R'_5$ einen Pyrrolidin-, Piperidin-, Hexamethylenimin- oder Morpholinrest,

$R'_5$ Alkyl mit 1 bis 4 C-Atomen oder zusammen mit $R'_4$ einen Pyrrolidin-, Piperidin-, Hexamethylenimin- oder Morpholinrest,

$R_{11}$ Wasser, Chlor, Alkyl mit 1 bis 4 C-Atomen oder Alkoxy mit 1 bis 4 C-Atomen,

n die Zahl 1 oder 2 und

M' -Alkylen $N(R'_4R'_5)$ oder den Rest

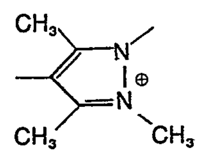

bedeuten.

4. Kationische Aufheller gemäss Anspruch 3 der Formel

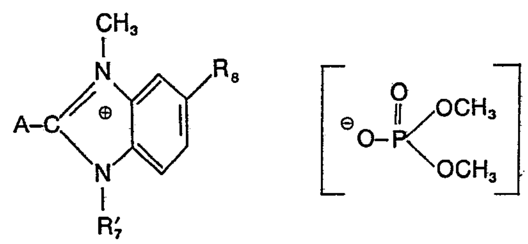

worin A einen Rest der Formel

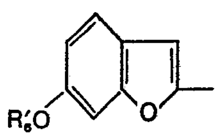

oder

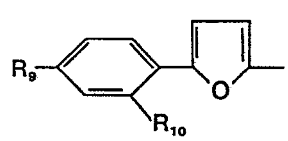

wobei $R'_6$ für Alkyl mit 1 bis 4 C-Atomen und $R_9$ und $R_{10}$ unabhängig voneinander für Wasserstoff, Chlor oder Methyl stehen, $R'_7$ Alkyl mit 1 bis 4 C-Atomen, Benzyl, Phenyl, Alkenyl mit 3 oder 4 C-Atomen, Cyanoalkyl mit 2 oder 3 C-Atomen, Alkoxycarbonylmethyl mit 1 bis 3 C-Atomen im Alkoxyteil und $R_8$ Wasserstoff, Methyl, Chlor, Alkylsulfonyl mit 1 bis 4 C-Atomen, Phenylsulfonyl, Cyano, Trifluormethyl, Phenoxysulfonyl, Alkoxycarbonyl mit insgesamt 2 bis 5 C-Atomen, Carboxy, $CONZ'_1Z'_2$, oder $SO_2NZ'_2$, worin $Z'_1$ für Wasserstoff, Alkyl mit 1 bis 4 C-Atomen, Alkenyl mit 3 oder 4 C-Atomen, Hydroxyalkyl mit 2 bis 4 C-Atomen, Alkoxyalkyl mit insgesamt 3 bis 6 C-Atomen oder Benzyl und $Z'_2$ für Wasserstoff, Alkyl mit 1 bis 4 C-Atomen stehen oder $Z'_1$ und $Z'_2$ zusammen mit dem Stickstoffatom auch einen Morpholinring bilden können, bedeuten.

5. Kationische Aufheller gemäss Anspruch 3 der Formel

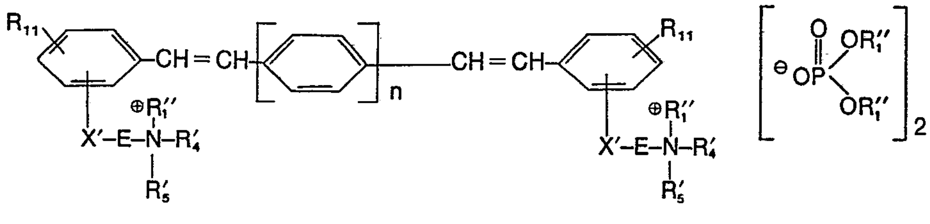

worin $R''_1$ Alkyl mit 1 bis 3 C-Atomen, X' Sauerstoff, Schwefel, die direkte Bindung $-COO-$, $-CON(R'')-$ oder $-SO_2N(R'')-$, R'' Wasserstoff, Alkyl mit 1 bis 4 C-Atomen oder zusammen mit $R'_4$ einen Piperazinrest, E unsubstituiertes oder durch eine OH-Gruppe substituiertes Alkylen mit 1 bis 4 C-Atomen, $R'_4$ Alkyl mit 1 bis 4 C-Atomen, zusammen mit R'' einen Piperazinrest oder zusammen mit $R'_5$ einen Pyrrolidin-, Piperidin-, Hexamethylenimin- oder Morpholinrest, $R'_5$ Alkyl mit 1 bis 4 C-Atomen oder zusammen mit $R'_4$ einen Pyrrolidin-, Piperidin-, Hexamethylenimin- oder Morpholinrest, $R_{11}$ Wasserstoff, Chlor, Alkyl mit 1 bis 4 C-Atomen oder Alkoxy mit 1 bis 4 C-Atomen und n die Zahl 1 oder 2 bedeuten.

6. Kationische Aufheller gemäss Anspruch 4 der Formel

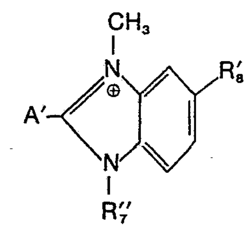

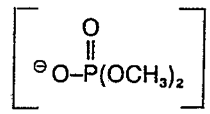

worin A′ einen Rest der Formel

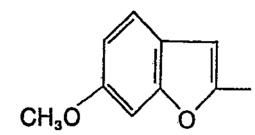

oder

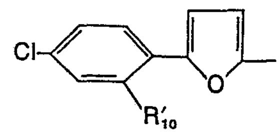

R″₇ Alkyl mit 1 bis 4 C-Atomen, Benzyl, Phenyl oder Cyanoalkyl mit 2 bis 3 Kohlenstoffatomen, $R'_8$ Wasserstoff, Alkylsulfonyl mit 1 bis 4 Kohlenstoffatomen, Phenylsulfonyl, Phenoxysulfonyl, Sulfamoyl, Alkylsulfamoyl mit 1 bis 4 Kohlenstoffatomen, Alkoxycarbonyl mit 2 bis 4 Kohlenstoffatomen, Cyano oder Chlor und $R'_{10}$ Wasserstoff, Chlor oder Methyl bedeuten.

7. Kationische Aufheller gemäss Anspruch 5 der Formel

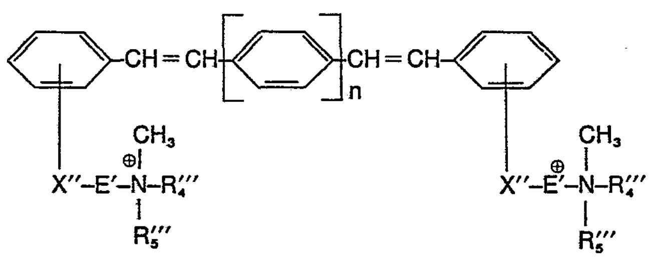

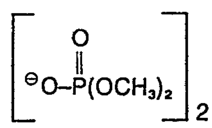

worin X″ Sauerstoff, die direkte Bindung, $-SO_2NH-$ oder $-CONH-$ in 2- oder 3-Stellung, E′ Alkylen mit 1 bis 3 C-Atomen, $R'''_4$ und $R'''_5$ Alkyl mit 1 oder 2 C-Atomen oder zusammen einen Pyrrolidin-, Piperidin- oder Morpholinrest und n die Zahl 1 oder 2 bedeuten.

8. Verfahren zur Herstellung von kationischen Aufhellern der in Anspruch 1 definierten Formel

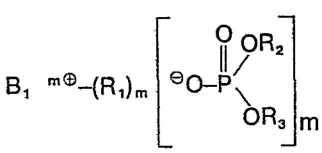

worin $B_1$, $R_1$, $R_2$, $R_3$ und m die in Anspruch 1 angegebene Bedeutung haben, dadurch gekennzeichnet, dass ein Aufheller $B_1$ mit mindestens stöchiometrischen Mengen m eines Phosphates der Formel

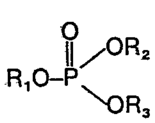

worin $R_1$, $R_2$ und $R_3$ die oben angegebene Bedeutung haben, bei Temperaturen zwischen 60 und 200 °C quaterniert.

9. Verfahren zum optischen Aufhellen von organischen Materialien, dadurch gekennzeichnet, dass man Verbindungen wie in einem der Ansprüche 1 bis 7 definiert, diesen Materialien einverleibt oder auf deren Oberfläche aufbringt.

10. Verfahren gemäss Anspruch 9 zum optischen Aufhellen von Polyacrylnitril, Polyamid und Cellulose.

11. Verfahren gemäss Anspruch 10 zum optischen Aufhellen von Polyacrylnitrilfasern im Gelzustand.

12. Konzentrierte wässrige Aufhellerlösung mit einem Gehalt von 1–60 Gew.%, bezogen auf das Gewicht der Lösung, eines kationischen Aufhellers wie in einem der Ansprüche 1 bis 7 definiert und gegebenenfalls einem überschüssigen wasserlöslichen Trialkyl- oder Trialkenylphosphat.

13. Konzentrierte wässrige Aufhellerlösung gemäss Anspruch 12, welche noch einen Puffer enthält.

## Claims

1. A cationic fluorescent whitening agent of the formula

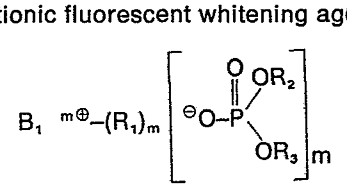

wherein $B_1$ is a fluorescent whitening agent belonging to the group of the 2-furanylbenzimidazoles, 2-azolylbenzimidazoles, 2-stilbenylbenzimidazoles, basic 1,2-bis-(azolyl)ethylenes, 2,5-(benzimidazolyl)furanes, basic 4,4′-bis-(azolyl)-stilbenes, basic 2-phenyl-5-azolyl-thiophenes, basic 4,4′-distyrylbiphenyls, basic 1,4-distyrylbenze-

nes, basic 3,7-disubstituted coumarins, basic naphthalimides, basic 4,4'-triazinylaminostilbenes, basic 2-stilben-4-yl-naphthotriazoles and basic triazolyl- or pyrazolylstilbenes, m is the number of basic amino groups, and each of $R_1$, $R_2$ and $R_3$ is alkyl of 1 to 4 carbon atoms which is unsubstituted or substituted by non-chromophoric groups, or is alkenyl of 2 to 4 carbon atoms which is unsubstituted or substituted by non-chromophoric groups.

2. A cationic fluorescent whitening agent according to claim 1 of the formula

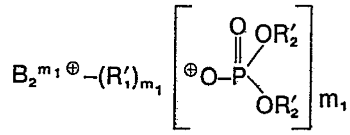

wherein $m_1$ is 1 or 2 and each of $R'_1$ and $R'_2$ is alkyl of 1 to 4 carbon atoms or benzyl, and $B_2$ is a fluorescent whitening agent of the formula

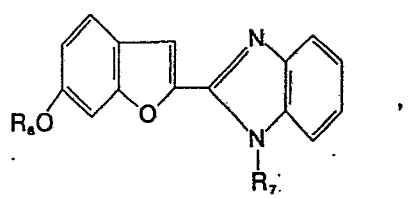
,
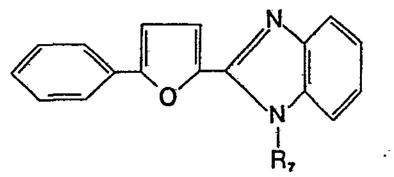
,
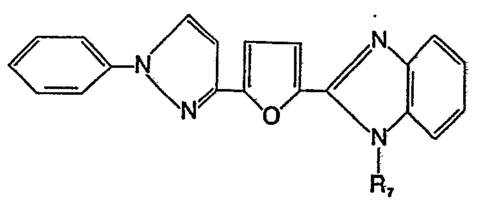
,
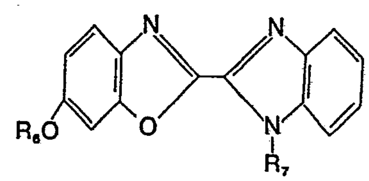
,
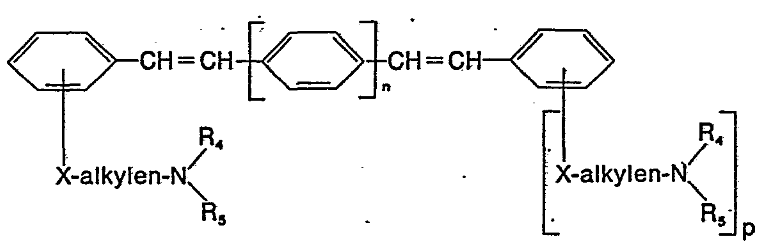

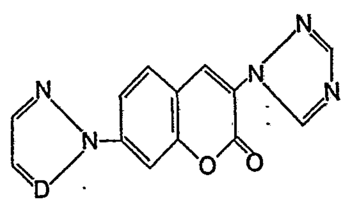
or
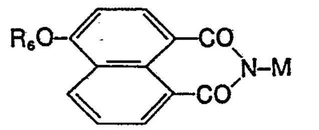

wherein M is alkylene-$N(R_4)(R_5)$, pyrazolyl or pyridinyl, each of $R_4$ and $R_5$ independently is alkyl of 1 to 6 carbon atoms which is unsubstituted or substituted by non-chromophoric groups, alkenyl of 2 to 6 carbon atoms which is unsubstituted or substituted by non-chromophoric groups, or $R_4$ and $R_5$, together with the nitrogen atom to which they are attached, also from a 5- to 7-membered heterocyclic ring, each of $R_6$ and $R_7$ independently is alkyl of 1 to 6 carbon atoms which is unsubstituted or substituted by non-chromophoric groups, alkenyl or 2 to 6 carbon atoms which is unsubstituted or substituted by non-chromophoric groups, or is phenyl, D is nitrogen or the –CH– group, X is a non-chromophoric bridge member or the direct bond, n is 1 or 2, and p is 0 or 1, and the benzene rings and the heterocyclic rings can also carry non-chromophoric substituents.

3. A cationic fluorescent whitening agent according to claim 2 of the formula

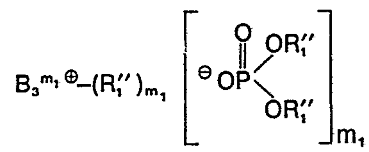

wherein $m_1$ is 1 or 2 and $R''_1$ is alkyl of 1 to 3 carbon atoms and $B_3$ is a fluorescent whitening agent radical of the formula

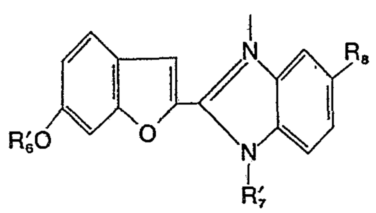
,

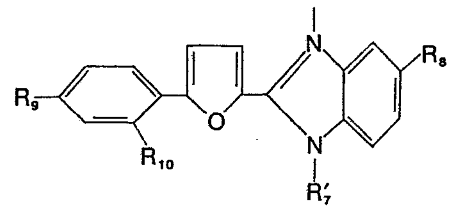
,

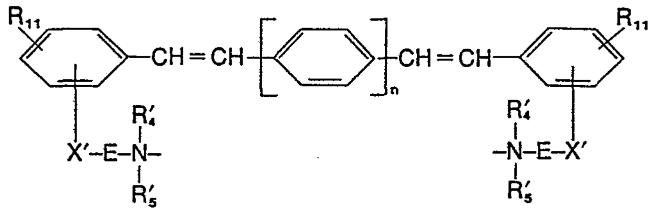

or

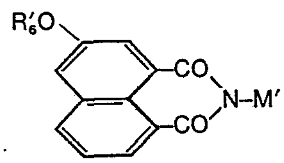
.

in which radicals

$R'_6$ is alkyl of 1 to 4 carbon atoms,

$R'_7$ is alkyl of 1 to 4 carbon atoms, benzyl, phenyl, alkenyl of 3 to 4 carbon atoms, cyanoalkyl of 2 or 3 carbon atoms, alkoxycarbonylmethyl containing 1 to 3 carbon atoms in the alkoxy moiety,

$R_8$ is hydrogen, methyl, chlorine, alkylsulfonyl of 1 to 4 carbon atoms, phenylsulfonyl, cyano, trifluoromethyl, phenoxysulfonyl, alkoxycarbonyl containing a total of 2 to 5 carbon atoms, carboxyl, $-CONZ'_1Z'_2$ or $-SO_2NZ'_1Z'_2$, wherein $Z'_1$ is hydrogen, alkyl of 1 to 4 carbon atoms, alkenyl of 3 or 4 carbon atoms, hydroxyalkyl of 2 to 4 carbon atoms, alkoxyalkyl containing a total of 3 to 6 carbon atoms, or benzyl, and $Z'_2$ is hydrogen, alkyl of 1 to 4 carbon atoms, or $Z'_1$ and $Z'_2$, together with the nitrogen atom to which they are attached, can also form a morpholine ring,

$R_9$ and $R_{10}$, each independently of the other, are hydrogen, chlorine or methyl,

R′ is hydrogen, alkyl of 1 to 4 carbon atoms which is unsubstituted or substituted by a cyano group, or together with $R'_4$ is a piperazine radical,

X′ is oxygen, sulfur, the direct bond, $-SO_2NR'-$, $-CONR'$ or $-COO-$,

E is alkylene of 1 to 4 carbon atoms which is unsubstituted or substituted by a –OH group,

$R'_4$ is alkyl of 1 to 4 carbon atoms, or together with R′ is a piperazine radical or together with $R'_5$ is a pyrrolidine, piperidine, hexamethyleneimine or morpholine radical,

$R'_5$ is alkyl of 1 to 4 carbon atoms, or together with $R'_4$ is a pyrrolidine, piperidine, hexamethyleneimine or morpholine radical,

$R_{11}$ is hydrogen, chlorine, alkyl of 1 to 4 carbon atoms or alkoxy of 1 to 4 carbon atoms,

n is 1 or 2, and

M′ is -alkylene-$N(R'_4R'_5)$ or the radical

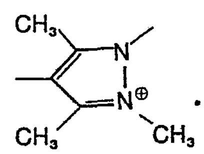
.

4. A cationic fluorescent whitening agent according to claim 3 of the formula

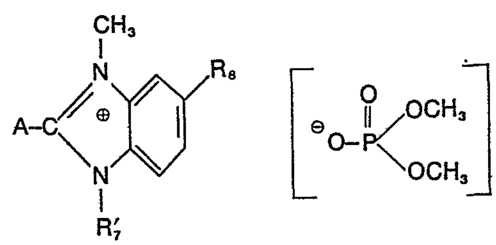
,

wherein A is a radical of the formula

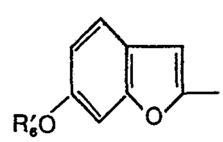
or
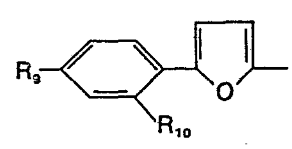

wherein $R'_6$ is alkyl of 1 to 4 carbon atoms and each of $R_9$ and $R_{10}$ independently is hydrogen, chlorine or methyl, $R'_7$ is alkyl of 1 to 4 carbon atoms, benzyl, phenyl, alkenyl of 3 or 4 carbon atoms, cyanoalkyl of 2 or 3 carbon atoms, alkoxycarbonylmethyl containing 1 to 3 carbon atoms in the alkoxy moiety, and $R_8$ is hydrogen, methyl, chlorine, alkylsulfonyl of 1 to 4 carbon atoms, phenylsulfonyl, cyano, trifluoromethyl, phenoxysulfonyl, alkoxycarbonyl containing a total of 2 to 5 carbon atoms, carboxyl, $CONZ'_1Z'_2$ or $SO_2NZ'_1Z'_2$, wherein $Z'_1$ is hydrogen, alkyl of 1 to 4 carbon atoms, alkenyl of 3 or 4 carbon atoms, hydroxyalkyl of 2 to 4 carbon atoms, alkoxyalkyl containing a total of 3 to 6 carbon atoms, or benzyl, and $Z'_2$ is hydrogen, alkyl of 1 to 4 carbon atoms, or $Z'_1$ and $Z'_2$, together with the nitrogen atom to which they are attached, can also form a morpholine ring.

5. A cationic fluorescent whitening agent according to claim 3 of the formula

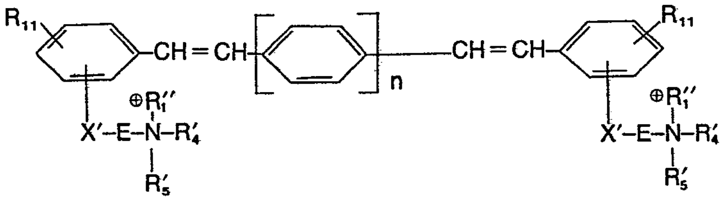

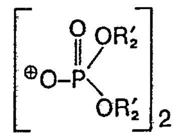

wherein $R''_1$ is alkyl of 1 to 3 carbon atoms, X' is oxygen, sulfur, the direct bond, –COO–, –CON(R'')– or $-SO_2N(R'')-$, R'' is hydrogen, alkyl of 1 to 4 carbon atoms, or together with $R'_4$ is a piperazine radical, E is alkylene of 1 to 4 carbon atoms which is unsubstituted or substituted by an –OH group, $R'_4$ is alkyl of 1 to 4 carbon atoms, or together with R'' is a piperazine radical or together with $R'_5$ is a pyrrolidine, piperidine, hexamethyleneimine or morpholine radical, $R'_5$ is alkyl of 1 to 4 carbon atoms or together with $R'_4$ is a pyrrolidine, piperidine, hexamethyleneimine or morpholine radical, $R_{11}$ is hydrogen, chlorine, alkyl of 1 to 4 carbon atoms or alkoxy of 1 to 4 carbon atoms, and n is 1 or 2.

6. A cationic fluorescent whitening agent according to claim 4 of the formula

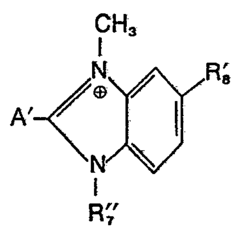

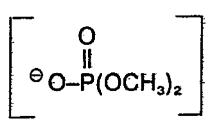

wherein A' is a radical of the formula

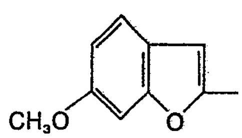

or

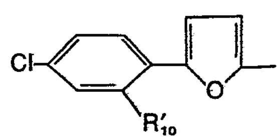

$R''_7$ is alkyl of 1 to 4 carbon atoms, benzyl, phenyl, or cyanoalkyl of 2 to 3 carbon atoms, $R'_8$ is hydrogen, alkylsulfonyl of 1 to 4 carbon atoms, phenylsulfonyl, phenoxysulfonyl, sulfamoyl, alkylsulfamoyl of 1 to 4 carbon atoms, alkoxycarbonyl of 2 to 4 carbon atoms, cyano or chlorine, and $R'_{10}$ is hydrogen, chlorine or methyl.

7. A cationic fluorescent whitening agent according to claim 5 of the formula

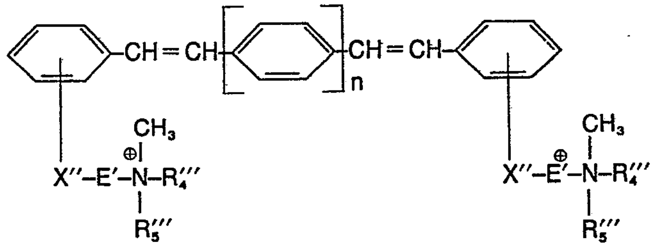

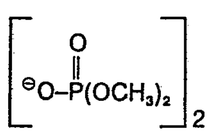

wherein X'' is oxygen, the direct bond, $-SO_2NH-$ or –CONH– in the 2- or 3-position, E' is alkylene of 1 to 3 carbon atoms, $R'''_4$ and $R'''_5$ are alkyl of 1 or 2 carbon atoms, or together are a pyrrolidine, piperidine or morpholine radical, and n is 1 or 2.

8. A process for the production of a cationic fluorescent whitening agent of the formula

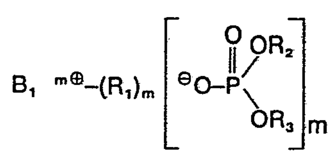

as defined in claim 1, wherein $B_1$, $R_1$, $R_2$, $R_3$ and

m are as defined in claim 1, which process comprises quaternising a fluorescent whitening agent $B_1$ with at least stoichiometric amounts m of a phosphate of the formula

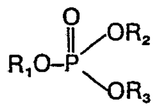

wherein $R_1$, $R_2$ and $R_3$ are as defined above, in the temperature range from 60° to 200 °C.

9. A process for whitening organic material, which comprises incorporating in said material or applying thereto a compound as defined in any one of claims 1 to 7.

10. A process according to claim 9, wherein the organic material is polyacrylonitrile, polyamide and cellulose.

11. A process according to claim 10, which comprises whitening polyacrylonitrile fibres in the gel state.

12. A concentrated aqueous solution having a content of 1 to 60% by weight, based on the weight of said solution, of a cationic fluorescent whitening agent as defined in any one of claims 1 to 7, and, optionally, an excess of a water-soluble trialkyl- or trialkenylphosphate.

13. A concentrated aqueous solution according to claim 12, which additionally contains a buffer.

**Revendications**

1. Agents de blanchiment optique cationiques de formule:

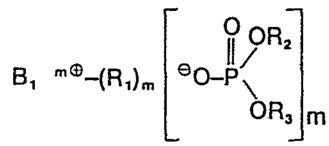

dans laquelle $B_1$ est un azurant optique appartenant au groupe des 2-furanyl-benzimidazoles, 2-azolyl-benzimidazoles, 2-stilbènyl-benzimidazoles, 1,2-bis-(azolyl)-éthylènes baziques, 2,5-bis-(benzimidazolyl)-furannes, 4,4'-bis-(azolyl)-stilbènes basiques, 2-phényl-5-azolyl-thiophènes basiques, 4,4'-distyrylbiphényles basiques, 1,4-distyryl-benzènes basiques, coumarines basiques disubstitués en 3,7, naphthalimides basiques, 4,4'-triazinylamino-stilbènes basiques, 2-stilbèn-4-yl-naphtotriazoles basiques et triazolyl- ou pyrazolyl-stilbènes basiques; m est le nombre de groupes amino basiques, et $R_1$, $R_2$ et $R_3$ sont des groupes alkyle ayant 1 à 4 atomes de carbone, non substitués ou portant un substituant non-chromophore ou bien des groupes alcényles ayant 2 à 4 atomes de carbone non substitués ou portant un substituant non-chromophore.

2. Agents de blanchiment optique cationiques selon la revendication 1, de formule:

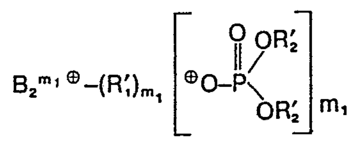

dans laquelle $m_1$ vaut 1 ou 2, et $R'_1$ et $R'_2$ sont des groupes alkyle ayant 1 à 4 atomes de carbone ou des groupes benzyle, et $B_2$ est un azurant de formules:

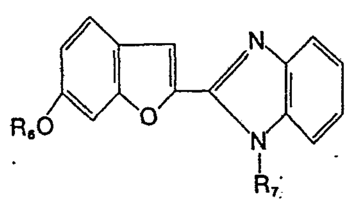

,

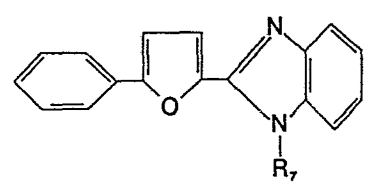

,

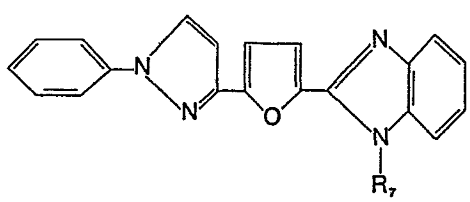

,

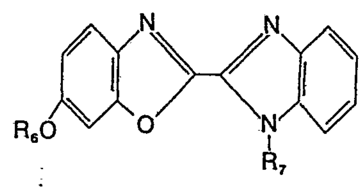

,

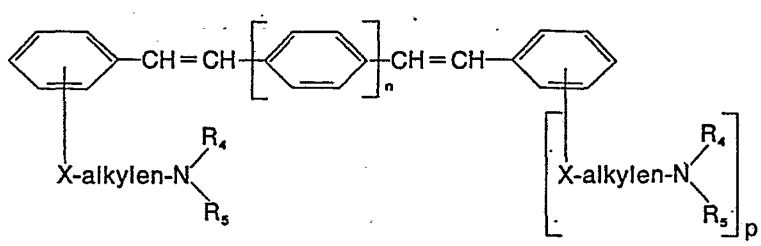

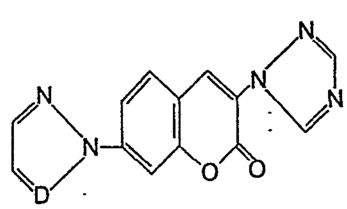

ou

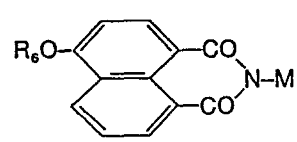

dans lesquelles M est un groupe alkylène-$N(R_4)(R_5)$, pyrazolyle ou pyridinyle; $R_4$ et $R_5$ indépendamment l'un de l'autre sont des groupes alkyle ayant 1 à 6 atomes de carbone non substitués ou portant un substituant non-chromophore, des groupes alcényle ayant 2 à 6 atomes de carbone non substitués ou portant un substituant non-chromophore, ou bien $R_4$ et $R_5$ ensemble avec l'atome d'azote forment également un noyau hétérocyclique de 5 à 7 chaînons; $R_6$ et $R_7$ indépendamment l'un de l'autre sont des groupes alkyle ayant 1 à 6 atomes de carbone non substitués ou portant un substituant non-chromophore, des groupes alcényle ayant 2 à 6 atomes de carbone non substitués ou portant un substituant non-chromophore, ou bien des groupes phényle; D est un atome d'azote ou le groupe CH; X est un chaînon pontal non-chromophore ou bien la liaison directe; n est le nombre 1 ou 2, et p est le nombre 0 ou 1, les noyaux benzéniques et les noyaux hétérocycliques pouvant porter également des substituants non-chromophores.

3. Agents de blanchiment optique cationiques selon la revendication 2, ayant la formule:

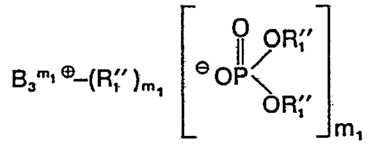

dans laquelle $m_1$ vaut 1 ou 2, et $R''_1$ est un groupe alkyle ayant 1 à 3 atomes de carbone, et $B_3$ est un reste d'azurant de formules:

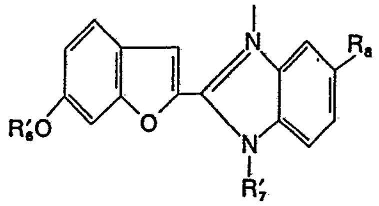

,

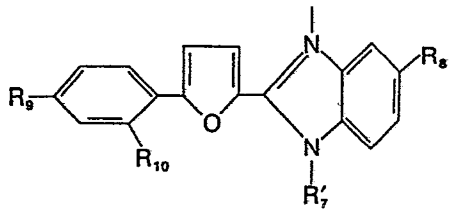

,

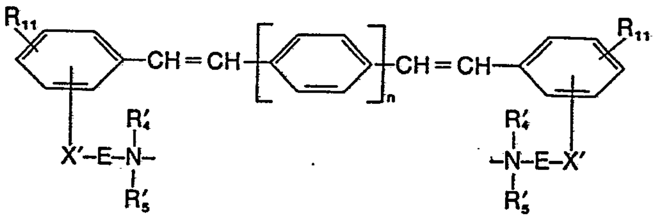

ou

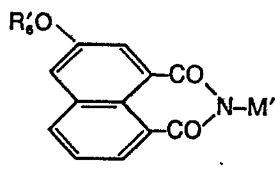

restes dans lesquels

$R'_6$ est un groupe alkyle ayant 1 à 4 atomes de carbone,

$R'_7$ est un groupe alkyle ayant 1 à 4 atomes de carbone, benzyle, phényle, alcényle ayant 3 ou 4 atomes de carbone, cyanoalkyle ayant 2 ou 3 atomes de carbone, alcoxycarbonylméthyle ayant 1 à 3 atomes de carbone dans la partie alcoxy,

$R_8$ est un atome d'hydrogène, un groupe méthyle, chloroalkylsulfonyle ayant 1 à 4 atomes de carbone, phénylsulfonyle, cyano, trifluorométhyle, phénoxysulfonyle, alcoxycarbonyle ayant en tout 2 à 5 atomes de carbone, carboxyle, $-CONZ'_1Z'_2$ ou $-SO_2NZ'_1Z'_2$, dans lesquels $Z'_1$ est un atome d'hydrogène, un groupe alkyle ayant 1 à 4 atomes de carbone, alcényle ayant 3 ou 4 atomes de carbone, hydroxyalkyle ayant 2 à 4 atomes de carbone, alcoxyalkyle ayant en tout 3 à 6 atomes de carbone ou bien un groupe benzyle, et $Z'_2$ est un atome d'hydrogène, un groupe alkyle ayant 1 à 4 atomes de carbone, ou bien $Z'_1$ et $Z'_2$ ensemble avec l'atome d'azote peuvent former également un noyau morpholine;

$R_9$ et $R_{10}$ indépendamment l'un de l'autre sont des atomes d'hydrogène, de chlore ou des groupes méthyle,

R' est un atome d'hydrogène, un groupe alkyle ayant 1 à 4 atomes de carbone non substitué ou substitué par un radical cyano ou bien forme conjointement à $R'_4$ un reste pipérazine;

X' est un atome d'oxygène, de soufre, la liaison directe $-SO_2NR'-$, $-CONR'-$, ou bien $-COO-$;

E est un groupe alkylène ayant 1 à 4 atomes de carbone non substitué ou substitué par un radical $-OH$;

$R'_4$ est un groupe alkyle ayant 1 à 4 atomes de

carbone ou forme conjointement à R′ un reste pipérazine ou bien forme conjointement à $R'_5$ un reste pyrrolidine, pipéridine, hexaméthylène-imine ou morpholine;

$R'_5$ est un groupe alkyle ayant 1 à 4 atomes de carbone ou bien forme conjointement à $R'_4$ un reste pyrrolidine, pipéridine, hexaméthylène-imine ou morpholine;

$R_{11}$ est un atome d'hydrogène, de chlore, un groupe alkyle ayant 1 à 4 atomes de carbone ou alcoxy ayant 1 à 4 atomes de carbone;

n est le nombre 1 ou 2, et

M′ est un groupe alkylène-N($R'_4R'_5$) ou le reste

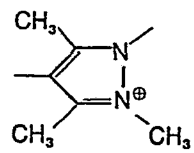

4. Agents de blanchiment optique cationiques selon la revendication 3, de formule:

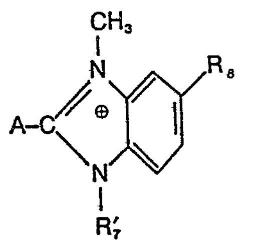

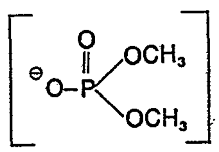

,

dans laquelle A est un reste de formules:

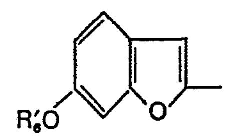

ou

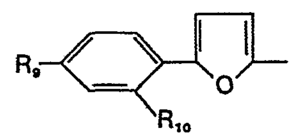

dans lesquelles $R'_6$ est un groupe alkyle ayant 1 à 4 atomes de carbone et $R_9$ et $R_{10}$, indépendamment l'un de l'autre, sont des atomes d'hydrogène, de chlore ou des groupes méthyle; $R'_7$ est un groupe alkyle ayant 1 à 4 atomes de carbone, benzyle, phényle, alcényle, ayant 3 ou 4 atomes de carbone, cyanoalkyle ayant 2 ou 3 atomes de carbone, alcoxycarbonylméthyle ayant 1 à 3 atomes de carbone dans la partie alcoxy, et $R_8$ est un atome d'hydrogène, de chlore, un groupe méthyle, alkylsulfonyle ayant 1 à 4 atomes de carbone, phénylsulfonyle, cyano, trifluorométhyle, phénoxysulfonyle, alcoxycarbonyle ayant en tout 2 à 5 atomes de carbone, carboxyle, $CONZ'_1Z'_2$ ou $SO_2NZ'_1Z'_2$, dans lesquels $Z'_1$ est un atome d'hydrogène, un groupe alkyle ayant 1 à 4 atomes de carbone, alcényle ayant 3 ou 4 atomes de carbone, hydroxyalkyle ayant 2 à 4 atomes de carbone, alcoxyalkyle ayant 3 à 6 atomes de carbone ou benzyle, et $Z'_2$ est un atome d'hydrogène, un groupe alkyle ayant 1 à 4 atomes de carbone, ou bien $Z'_1$ et $Z'_2$ peuvent former ensemble avec l'atome d'azote également un noyau morpholine.

5. Agents de blanchiment optique cationiques selon la revendication 3, ayant la formule:

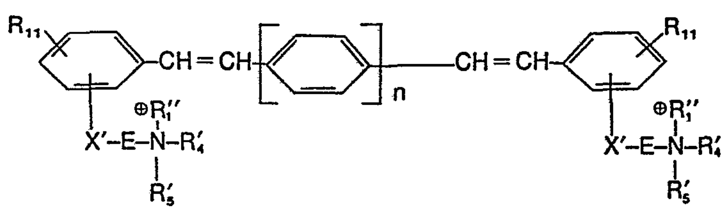

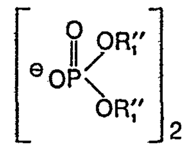

dans laquelle $R''_1$ est un groupe alkyle ayant 1 à 3 atomes de carbone; X′ est un atome d'oxygène, de soufre, la liaison directe, –COO–, –CON(R″)– ou bien –$SO_2$N(R″)–, R″ étant un atome d'hydrogène, un groupe alkyle ayant 1 à 4 atomes de carbone ou formant conjointement à $R'_4$ un reste pipérazine; E est un groupe alkylène ayant 1 à 4 atomes de carbone non substitué ou substitué par un radical –OH; $R'_4$ est un groupe alkyle ayant 1 à 4 atomes de carbone ou forme conjointement à R″ un reste pipérazine ou bien forme conjointement à $R'_5$ un noyau pyrrolidine, pipéridine, hexaméthylène-imine ou morpholine; $R'_5$ est un groupe alkyle ayant 1 à 4 atomes de carbone ou forme conjointement à $R'_4$ un reste pyrrolidine, pipéridine, hexaméthylène-imine ou morpholine; $R_{11}$ est un atome d'hydrogène, de chlore, un groupe alkyle ayant 1 à 4 atomes de carbone ou alcoxy ayant 1 à 4 atomes de carbone et n est le nombre 1 ou 2.

6. Agents de blanchiment optique cationiques selon la revendication 4, ayant la formule:

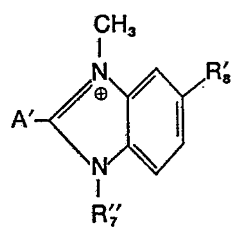

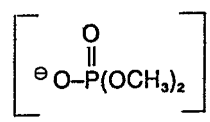

dans laquelle A′ est un reste de formules:

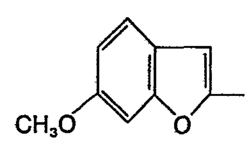

ou

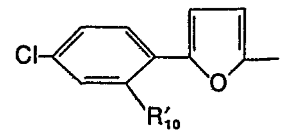

dans lesquelles $R''_7$ est un groupe alkyle ayant 1 à 4 atomes de carbone, benzyle, phényle ou cyanoalkyle ayant 2 ou 3 atomes de carbone; $R'_8$ est un atome d'hydrogène, un groupe alkylsulfonyle

ayant 1 à 4 atomes de carbone, phénylsulfonyle, phénoxysulfonyle, sulfamoyle, alkylsulfamoyle ayant 1 à 4 atomes de carbone, alcoxycarbonyle ayant 2 à 4 atomes de carbone, cyano ou chloro, et $R'_{10}$ est un atome d'hydrogène, de chlore ou le groupe méthyle.

7. Agents de blanchiment optique cationiques selon la revendication 5, ayant la formule:

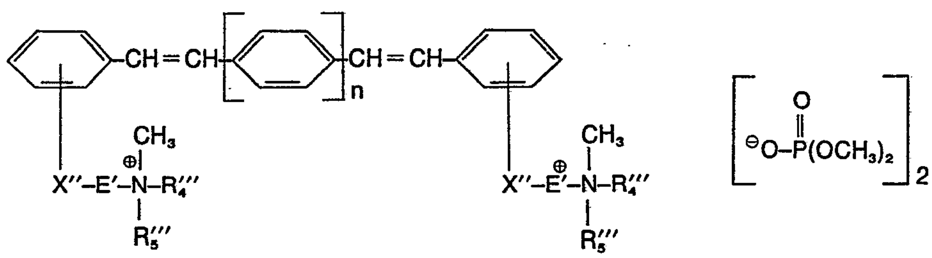

dans laquelle X″ est un atome d'oxygène, la liaison directe, $-SO_2NH-$ ou $-CONH-$ sur les positions 2 ou 3; E′ est un groupe alkylène ayant 1 à 3 atomes de carbone; $R'''_4$ et $R'''_5$ sont des groupes alkyle ayant 1 ou 2 atomes de carbone, ou bien forment ensemble un reste pyrrolidine, pipéridine ou morpholine, et n est le nombre 1 ou 2.

8. Procédé pour la préparation d'agents de blanchiment optique cationiques ayant la formule définie dans la revendication 1:

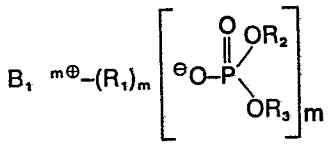

dans laquelle $B_1$, $R_1$, $R_2$, $R_3$ et m ont les significations données dans la revendication 1, caractérisé par le fait qu'on quaternise un azurant $B_1$ avec une quantité au moins stoechiométrique m d'un phosphate ayant la formule:

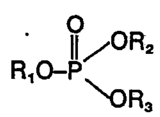

dans laquelle $R_1$, $R_2$ et $R_3$ ont les significations données ci-dessus, à des températures comprises entre 60 et 200 °C.

9. Procédé pour le blanchiment optique de matières organiques, caractérisé par le fait qu'on incorpore à ces matières ou qu'on dépose sur leur surface des composés tels que définis dans l'une quelconque des revendications 1 à 7.

10. Procédé selon la revendication 9, pour le blanchiment optique de polyacrylonitrile, de polyamide et de cellulose.

11. Procédé selon la revendication 10, pour le blanchiment optique de fibres en polyacrylonitrile à l'état de gel.

12. Solution aqueuse concentrée d'agents de blanchiment optique ayant une teneur pondérale de 1 à 60% par rapport au poids de la solution, d'un agent de blanchiment optique cationique tel que défini dans l'une quelconque des revendications 1 à 7 et contenant éventuellement un excès de trialkylphosphate ou de trialcénylphosphate dissous dans l'eau.

13. Solution aqueuse concentrée d'agents de blanchiment optique selon la revendication 12, qui contient encore un tampon.